# EUROPEAN PATENT APPLICATION

(11) **EP 2 093 289 A1**
(43) Date of publication of application: **26.08.2009**
(21) Application number: 07831987.8
(22) Date of filing: 16.11.2007
(51) Int. Cl.: C12N 15/00, A01K 67/027, A61K 45/00, A61P 25/22, C12N 15/09, G01N 33/15, G01N 33/50

(54) **KNOCKOUT ANIMAL EXHIBITING ANXIETY-LIKE BEHAVIOR**

(30) Priority: 17.11.2006 JP 2006312039
(71) Applicant: Hashimoto, Tamotsu, Kyoto-shi, Kyoto 606-8202 (JP)
(72) Inventor: TSUJIMURA, Atsushi, Muko-shi Kyoto 617-0001 (JP); HASHIMOTO, Tamotsu, Kyoto-shi, Kyoto 6068202 (JP)
(74) Representative: HOFFMANN EITLE
(86) International application number: PCT/JP2007/072257
(87) International publication number: WO 2008/059949

(57) **Abstract**

A vector for creating *kf-1* gene knockout nonhuman animals exhibiting increased anxiety-like behaviors, and containing Lox-pM-M-*kf-1*[in3b]-*kf-1*[ex4a]-LoxP, (wherein, M is a selection marker gene, pM is a promoter for the expression of the selection marker gene, *kf-1*[in3b] is a sequence represented by SEQ ID NO: 2, and *kf-1*[ex4a] is a sequence represented by SEQ ID NO: 3); *kf-1* gene knockout nonhuman animals exhibiting increased anxiety-like behaviors produced by using the vector or a descendant of the animal, and a method for use of the same.

SEQUENCE LISTING

<110>  HASHIMOTO, Tamotsu

<120>  KF-1 KO animal

<130>  P07-120

<150>  JP 2006-312039
<151>  2006-11-17

<160>  12

<170>  PatentIn version 3.3

<210>  1
<211>  1987
<212>  DNA
<213>  mouse

<400>  1
gtactgcaga aggagaggct gggtgcgttc cacactcatc atgtctgtgc cacaaacaag      60

tacatctaaa gggaaagtca tgcttaaaga gtacagtgga cgcaagattg aagtagagca     120

catttttaag tggataactg cccatgcagc ttctcggatc aaaactatat ataatgtgga     180

gcatttgaag gaagaatgga ataaaagtga tcaatactgg gtaaaaatat acctatttgc     240

aaaccttgac caaccgccag ctttcttctc tgcattaagt ataaaattta ctggaagagt     300

tgagtttatt tttgttaatg tggaaaattg gaacaacaag agttatatga cagatattgg     360

tatttataac atgccatcat acatacttag aactcctgaa ggaatttata gatacggaaa     420

ccacacaggt gaatttatat cccttcaggc catggattca tttctgcgct cattacaacc     480

tgaagtaaat gatctgtttg ttttgagttt ggttctagtt aatcttatgg cttggatgga     540

cttatttatt acacaaggag caaccatcaa gcgatttgtg gttctcataa gcactttagg     600

gacatacaat tccctattaa ttatttcttg gctacctgtg ttgggctttc tacagctccc     660

ttacttagat agcttttatg aatatagttt aagattgctg cgatattcta atacaaccac     720

actggcttcg tgggtaaggg cagactggat gttttactct tcacacccag ccctgtttct     780

cagtacatac cttggacatg gtttgctaat tgattacttt gagaagaaga gacgtcgcag     840

caacaatgat gaagttaatg cgaataattt agaatggtta tcaagtctgt gggactggta 900

caccagctac ctcttccacc cgattgcttc ttttcagaac tttcctgtag actctgattg 960

ggacgaagac cctgacttat tcttggaacg gttagctttc cctgaccttt ggcttcaccc 1020

tctgatacca actgattata ttaaaaactt accaatgtgg cgatttaaat gtcttggggt 1080

tcagtctgaa gaagaaatgt cagagagttc tcaagacact gaaaatgact cggatagtga 1140

caacatggac acttttagta gtagtaagga tatatttgaa gataaacaaa gcgttgttca 1200

cagttctcca ggaagaacaa gtcactgtga tactgaggct tgttcatgtg ccaataaatg 1260

tgagagcagc ccatgtgaaa ggaagaggag gtcgtatgga tcacataata ctgatgaaga 1320

tatggaaccg gactggttaa cttggcctgc gggtacgctg cactgtactg aatgtgttgt 1380

ttgccttgag aattttgaaa atggatgttt gctaatgggg ttgccttgtg gtcacgtgtt 1440

tcaccagaat tgcattgtta tgtggttggc tgggggccga cactgttgcc ctgtttgtcg 1500

ttggccttca tataagaaaa agcagccata tgcacaacaa cagccgttgt caaatgatgt 1560

tccatcttaa ccatgtgcaa tttgtcctta ataagccttg agtatcttac agcttgcctt 1620

tttaatgtta gtcacaatgt ttttgtggtt tgaagtttag tttaatgtta gtgcagtgac 1680

aggaaataca cattatgctg atgttgatga cagaatttat ttggttgcct tgtgtgtcaa 1740

ttgaatgcat actaaactgt aaaaaaaatt tatttacagc attgaaaatt cagaagttaa 1800

tggttttttg taagcacaaa agaagtatgg tagaaattaa tcttaacaag actttatgag 1860

gcaggatcaa atcctagtgg gcctgagcag gtttcttacc ctaagtgttt tctccctttt 1920

tacaatctct gtccagcacc tcttggttaa ataatgtatg ctctgagaca tgaaattaaa 1980

acagatc 1987

<210> 2
<211> 1680
<212> DNA
<213> mouse

<400> 2

atcaagctta tcgataccgt cgatcgacgg tatcgataag cttgatatcg aattcctgca 60

gccggccgca cgtctaagaa accattatta tcatgacatt aacctataaa aataggcgta 120

tcacgaggcc ctttcgtctt caagaattcc gatcatattc aataaccctt aatataactt 180

cgtataatgt atgctatacg aagttattag gtctgaagag gagtttacgt ccagccaagc 240

ttaggatcga tccgaacaaa cgacccaaca cccgtgcgtt ttattctgtc tttttattgc 300

cgatcccctc agaagaactc gtcaagaagg cgatagaagg cgatgcgctg cgaatcggga 360

gcggcgatac cgtaaagcac gaggaagcgg tcagcccatt cgccgccaag ctcttcagca 420

atatcacggg tagccaacgc tatgtcctga tagcggtccg ccacacccag ccggccacag 480

tcgatgaatc cagaaaagcg gccattttcc accatgatat tcggcaagca ggcatcgcca 540

tgggtcacga cgagatcctc gccgtcgggc atgcgcgcct tgagcctggc gaacagttcg 600

gctggcgcga gcccctgatg ctcttcgtcc agatcatcct gatcgacaag accggcttcc 660

atccgagtac gtgctcgctc gatgcgatgt ttcgcttggt ggtcgaatgg gcaggtagcc 720

ggatcaagcg tatgcagccg ccgcattgca tcagccatga tggatacttt ctcggcagga 780

gcaaggtgag atgacaggag atcctgcccc ggcacttcgc ccaatagcag ccagtccctt 840

cccgcttcag tgacaacgtc gagcacagct gcgcaaggaa cgcccgtcgt ggccagccac 900

gatagccgcg ctgcctcgtc ctgcagttca ttcagggcac cggacaggtc ggtcttgaca 960

aaaagaaccg ggcgcccctg cgctgacagc cggaacacgg cggcatcaga gcagccgatt 1020

gtctgttgtg cccagtcata gccgaatagc ctctccaccc aagcggccgg agaacctgcg 1080

tgcaatccat cttgttcaat ggccgatccc atattggctg caggtcgaaa ggcccggaga 1140

tgaggaagag gagacagcgc ggcagacgtg cgcttttgaa gcgtgcagaa tgccgggcct 1200

ccggaggacc ttcgggcgcc cgccccgccc ctgagcccgc ccctgagccc gcccccggac 1260

ccaccccttc ccagcctctg agcccagaaa gcgaaggagc caaagctgct attggccgct 1320

gccccaaagg cctacccgct tccattgctc agcggtgctg tccatctgca cgagactagt 1380

gagacgtgct acttccattt gtcacgtcct gcacgacgcg agctgcgggg cgggggggaa 1440

cttcctgact aggggaggag tagaaggtgg cgcgaagggg ccaccaaaga acggagccgg 1500

ttggcgccta ccggtggatg tggaatgtgt gcgaggccag aggccacttg tgtagcgcca 1560

agtgcccagc ggggctgcta aagcgcatgc tccagactgc cttgggaaaa gcgcctcccc 1620

tacccggtag aattgacctg caggggccct cgaggggtcg acggtatcga taagcttgat 1680

<210> 3
<211> 1987
<212> DNA
<213> mouse

<400> 3
gtactgcaga aggagaggct gggtgcgttc cacactcatc atgtctgtgc cacaaacaag 60

tacatctaaa gggaaagtca tgcttaaaga gtacagtgga cgcaagattg aagtagagca 120

catttttaag tggataactg cccatgcagc ttctcggatc aaaactatat ataatgtgga 180

gcatttgaag gaagaatgga ataaaagtga tcaatactgg gtaaaaatat acctatttgc 240

aaaccttgac caaccgccag ctttcttctc tgcattaagt ataaaattta ctggaagagt 300

tgagtttatt tttgttaatg tggaaaattg gaacaacaag agttatatga cagatattgg 360

tatttataac atgccatcat acatacttag aactcctgaa ggaatttata gatacggaaa 420

ccacacaggt gaatttatat cccttcaggc catggattca tttctgcgct cattacaacc 480

tgaagtaaat gatctgtttg ttttgagttt ggttctagtt aatcttatgg cttggatgga 540

cttatttatt acacaaggag caaccatcaa gcgatttgtg gttctcataa gcactttagg 600

gacatacaat tccctattaa ttatttcttg gctacctgtg ttgggctttc tacagctccc 660

ttacttagat agcttttatg aatatagttt aagattgctg cgatattcta atacaaccac 720

actggcttcg tgggtaaggg cagactggat gttttactct tcacacccag ccctgtttct 780

cagtacatac cttggacatg gtttgctaat tgattacttt gagaagaaga gacgtcgcag 840

caacaatgat gaagttaatg cgaataattt agaatggtta tcaagtctgt gggactggta 900

caccagctac ctcttccacc cgattgcttc ttttcagaac tttcctgtag actctgattg 960

ggacgaagac cctgacttat tcttggaacg gttagctttc cctgaccttt ggcttcaccc 1020

tctgatacca actgattata ttaaaaactt accaatgtgg cgatttaaat gtcttggggt 1080

tcagtctgaa gaagaaatgt cagagagttc tcaagacact gaaaatgact cggatagtga 1140

caacatggac acttttagta gtagtaagga tatatttgaa gataaacaaa gcgttgttca 1200

cagttctcca ggaagaacaa gtcactgtga tactgaggct tgttcatgtg ccaataaatg 1260

tgagagcagc ccatgtgaaa ggaagaggag gtcgtatgga tcacataata ctgatgaaga 1320

tatggaaccg gactggttaa cttggcctgc gggtacgctg cactgtactg aatgtgttgt 1380

ttgccttgag aattttgaaa atggatgttt gctaatgggg ttgccttgtg gtcacgtgtt 1440

tcaccagaat tgcattgtta tgtggttggc tgggggccga cactgttgcc ctgtttgtcg 1500

ttggccttca tataagaaaa agcagccata tgcacaacaa cagccgttgt caaatgatgt 1560

tccatcttaa ccatgtgcaa tttgtcctta ataagccttg agtatcttac agcttgcctt 1620

tttaatgtta gtcacaatgt ttttgtggtt tgaagtttag tttaatgtta gtgcagtgac 1680

aggaaataca cattatgctg atgttgatga cagaatttat ttggttgcct tgtgtgtcaa 1740

ttgaatgcat actaaactgt aaaaaaaatt tatttacagc attgaaaatt cagaagttaa 1800

tggttttttg taagcacaaa agaagtatgg tagaaattaa tcttaacaag actttatgag 1860

gcaggatcaa atcctagtgg gcctgagcag gtttcttacc ctaagtgttt tctccctttt 1920

tacaatctct gtccagcacc tcttggttaa ataatgtatg ctctgagaca tgaaattaaa 1980

acagatc 1987

<210> 4
<211> 31
<212> DNA
<213> mouse

<400> 4
gatctataaa ataaattatt ttaaaagcag a 31

<210> 5
<211> 10148
<212> DNA

<213>  mouse

<400>  5
   aggttgtagg tttctgatct taagctgtgg taaggtgact ggtttttgta ggtgttttgc      60
   tggattactg atttctcggg taattgcaac gtgattttga gatggaggga gaagaaatgg     120
   aatccttttg ctgacactgg ctatgagtaa tggtggtcta cctcacagcc taatatcttg     180
   gataggaaag aattttatag ggggctttga aaaatcagta cttagcatat tcatctaagt     240
   aaaaacaact ataacttttt agtcacacaa ggattacctt gtatgtttaa ctgacttttt     300
   attttgtaag tttttagtgg tagaggaaag ccgagcatat agatagtaga aaaaatatct     360
   ttctctttcg tgtaccatat ttgtatttta ttacatttac tttagcacct tttattacaa     420
   ttttaatact tcttcttttt ccctctcagt cagctgaaag taaacttgaa tcatgatgct     480
   ttgcccgtaa acagtcacca cattttctgt gaatgtcccc catacctctc cccccactcc     540
   cctacccacc cattcccact ttttggccct ggcgttcccc tgtactgggg catataaggt     600
   ttgcgtgtcc aatgggcctc tctttccagt gatggctgac taggccatct tttgatacat     660
   atgcagctag agacacgagc tccggggtac tggttagttc atattgttgt tccatctata     720
   ggattgcaga ccccattagc tccttgggta ctttctctag ctcctccatt gggggccctg     780
   tgatccatcc aatagctgac tgtgagcatc cacttctgtg tttgctaggc ccctgcatag     840
   tctcacaaga gacagctata tcagggtcct ttcagcaaac gcttgctagt gtatgcaatg     900
   gtgtcatcgt ttggaggcta attatgggat ggctccctgg atatggcagt ctctagatga     960
   tccatccttt tgtctcagct ccaaactttg tctctgttac tccttccatg ggtgattgtt    1020
   tccaattcta agaaggggca aagtgtccac actttggtct ttgttcttct tcagtttcgt    1080
   gtgttttaca aattgtctct tatatttcgg gtatactaag tttctgggct aatatccact    1140
   tatcagtgag tacatatcat ttgagttctt ttgtgattgt gttacctcac tcaggatgat    1200
   gccctccagg tccaaccatt tgcctaggaa tttcataaat tcattctttt taatagctga    1260
   gtagtgaacc cttgacattt aatacctaat atttgttcct tgtcccataa aagtctttat    1320
   atctctgtct ttgttttgtt gacttatctg ttgggtttag tttccctttc ttccatcttc    1380
   ccttagttcc tgtagagaaa ctgctgttca tgtgtgtgtg tgtgttgtgc taaggcttga 1440
   attcaggacc tcagcataat aggcaagtac cccaagctac accccagccc tctctaaatt 1500
   ttttaatttt gagatagtct cactagattg cctgagttgg tcttgaactc attatagctt 1560
   aggtaagcct tgagttctca atcttcctgc atcagcctct tagattacat gcctgagttg 1620
   caagcctgat gcctgttctg gtactaatat ttttaacagt acaaattggc ttatatagtg 1680
   ttccttgttt tctttcaaga ttaaatttga gtgctgctca tatacatcgt atcaggatgc 1740
   atgtagtgtc agttctgttg ttggtgattc gaaatgtgtt taatatctgt caagtctttc 1800
   ctttgtgaga taccttttcc ctctgtaatt taagaagcaa cctgtgattc tttgtgatct 1860
   tttccctcat ctcagtggtt ttaacagcca gtgatgattc tttgacttgg tcatcaagtt 1920
   agaatatggt cattttcaaa tttctgctta attgcacatt tattagctgg ctgtgtgtgt 1980
   gagagagagg cgaggagagc tttctatttt tctcccatgt tttgaatatc actggactta 2040
   gatgtttatc attattatta ttattattat tattattatt attattatta cccagtgtat 2100
   taaaacttta ggtgctagag agaatgctcg ttggttaaga gcactggcta ctcttacagg 2160
   agacctgggt ttgattccca gcatccatat gatagccaac tagccaccat cttatttgta 2220
   actacagttt caggaggtct aatacatttc ttgcctctgt aggcagtgaa cacacaaggt 2280
   acacaggcaa aacacccatg taagtaagta agtaagtaag taagaaagac agtcagtcag 2340
   tcagtcagtc ccaaagcact cataaaaatt attaaatttt aaaaaatgct tatttttggt 2400
   actcagtgtc atacttagct agtggaaacc tctttacact agccttatgt cttgttacta 2460
   atgtcttaat cctgaagcac ttgtttgctt tttgaccctg gtttcttgtc atcttttttt 2520
   cagagtaccc aagtttcatg atggacacct ctaagatcct tagttagctg cactccaaaa 2580
   tggaaatggg aaaaacaaaa tataaattat ttccaggata ctaacaattt tttcttgcat 2640
   gataaattgt gccttacttt caaatactaa tagaaaatag gtgctttttc acttttagtg 2700
   ggattatgtc ataataaatc catcataaat tgaaagtttc acaagtaaaa catgcattta 2760
   caagtctaaa gtcccaactt tacgtagaaa aatgtcggtt atccttcaag gtcctctgac 2820
   tggcagttcc agcttgatac ctctatccag aatactgtac tttcctaggc tgaacagtga 2880
   aattctagtc agaatttcaa ctaactacct ctaaagagac agacttcttt gtcatcatcc 2940
   taaaaattga gaaaccatca aacatcagca gaatgcttgt ctaaatagag aattcataaa 3000
   gccctgaatt ggtcctcagc accatataaa ctgacatggt ggtgcatacc tgtcgtccca 3060
   ctcagaggtg gaggatcaag ggtccagttc atgttcaact acatagagag ttcaaaacta 3120
   acctgagaga tattagacct tgtcttaaat aggaaaacac ctagtagaaa ggactagcag 3180
   ctatggattg tatttcataa atttgagaac ttaaaataac tgcagccatt gctgcatata 3240
   cttctgaaca agcgctgaaa tcctttcctt gtaaatggag tcccaagatt tctcacctct 3300
   tcttttgtcc atgctttcag cagatggttc tcaaatttac ctgttcttca tgggctcttt 3360
   ccacagcacc agttttatac acagatttct actagactcg cttgaagtac ttcggatcga 3420
   acttcggtct tttggggcat actaggcaag cactctccca tcctcagcct ttgatttttg 3480
   tagcttggga tggccttgaa ctcataatca tcctgaattc aattctgcca tgccctggct 3540
   ctgggttctt ttaattaact acagtgctct gttgtctttc ttcatcccca gtcttccttt 3600
   taaggtctta atggagatag gtggagttac aaaatgtaca tgaaagtgta gaaatgttta 3660
   gacaaaattt ctctagcttt ccagagcaca tcacacattg gatatcatta tcctaaacag 3720
   accttttgaa tcttttccat tcataatccc tttcattcaa gaaggtggct ttggggcatg 3780
   taggtatata aaatagctac acatatcaga catttactgg caataaaatt gtaaatatat 3840
   ttaaaagcct ctgctatccc tacagctttg ctgttcacta aagagtaaga cagtttttca 3900
   tactagtaag ataagtgtgc ttatttttac ataaagaatt aaatcttggg gttggagaga 3960
   tggctcaaca gttaagagca ctgactattc ttccagggct cttaagttca attctcagca 4020
   accacatagt ggctcacaac catctgtaat gggatccaat gcccggatct gttgtgtctg 4080
   aagacagcta cagtgtactc atgcacataa ataaatcttt aaaaaaaaag aattaaatct 4140
   tagccaagtg tagtggtaca cacctctaat ccaagggagg cagaggcagg tggatcttcc 4200
   tgagtttgag gccaacctgg tctataataa agtcccagaa cagacagggc tgttacataa 4260
   agaaaccctg ccttgaaaaa gtcaaaacaa ccccaaaata atcttgtctg aatattttat 4320
   gttagcctag aagcccagca catctttgac attatttaga actgatcaac atttttattt 4380
   ttatgattgt cagtgcagaa gatgcaactt tgtataaatc tgtaatataa aatgaaaaaa 4440
   aagcagaaat atgtttagag ccatttcaga aattgttgaa aattctgcct aatcccatgt 4500
   caaaattgat ttaacagttt ttaaggaaac tgagtcagca actggcagtt tttaaagaat 4560
   ctgatacaat ccagtctaaa cagttgctag ctatactagt atactgatat ggtagttaca 4620
   tgctaagaaa tgacaataaa gaaaaatttt aagcttttgc tttctgtgat taaaccatta 4680
   tgtttctgta agagcagaaa actgtatata cacttaaaat actgcagttc accacgtgat 4740
   agcgttgagt ctgagccaag atgtttcagg cagtttgtgt tggcactgtg tggcatccct 4800
   acacgtgaag tgcagcatgt gtgttgtatg ttcagagaca aggctgtggt gaagccaggc 4860
   tgtgaaacac aggtgaatgt taccagaaac accttagatt catcatctgt ctgattctgt 4920
   attacctttg ttcttttgca aattcaggaa gtttttactc ttaaaacatt tatccagacc 4980
   cccaaattta gttactggac caagttgagg actagacaac aaagaaatca gcattggcca 5040
   tcaatggtcc ctctcatttc tggcttacta tttaggctca ttttcatttc acattctttt 5100
   tttttaagac ttttatttat ttattatatg tgagtacact gtagctgtct tcagacactc 5160
   tggatgaggg tgtcagatct tgttacagat ggttgtgagc caccatgtgg ttgctgggat 5220
   ttgaactcag gacctttgga agagcagtcg atgttcttaa ccactgagcc atctcaccag 5280
   ccctcatttc acattcttac cacccgtgtt atttttcctc cggtgggtgc tttgttgtca 5340
   tcccctctac ctggttgtat tgttcctttt tctactcttt ctgttggctt ggttgaattt 5400
   tctccctaag ttctgcgcct tcccatcttc tgcttcctta ctagacttaa aagggggcaa 5460
   agatacttac aggtagtgac agtcagtgaa ggacacctta ttcaagtgat tcaactaatg 5520
   tcatcaacag ctgagtggag agatgtgtgc tctatgtttc aggtactttg gacattcctg 5580
   atagatgcat tgaactaaac attgcatgtg cctttttaga tcctttatgg tagggcctgt 5640
   gtgttattaa tctagctacc cacaatgcat ggtccaaaat ggacaaaata cttttttcag 5700
   tgttatgtgt gcacaaaaat tcctcagtga aaaaacaaaa ttacttcaaa atggttagag 5760
   ggactgccag ggtttcgggt cttcatacac gtggctcatc ccttaattgt taaatctgct 5820
   cttagcagat tttccctgag gtagagaact gaaggaaata ctgaagattg atagatttta 5880
   tttcaacttg tttttaaaga aaagttctgt ttctttgaac agtttcttgt atttaaggaa 5940
   ccccaagcat gtggtctttg caggtgcagg attccctgga taatatgaca cataatttgt 6000
   taggagactt ttgaaaccta aattattttg tgtgtaaaat ctcttcattc tgtaaagttg 6060
   ataagctccc tgaggggtga aatattatgt agaattgcct cctctcagta tgtaagatcc 6120
   tatatagata cagcaaataa ttgctggctt gaatcttaag aacatatttt agttttacct 6180
   ctctcccatc tcatgtaggt aaggaaggta aaagatgcta tttacttttt aggttacttg 6240
   ggagcctggc aagttattct tgtaaaatct ttctcttttc tcacctgtgt tttcatttta 6300
   ttattcctta aaactcagtc aaaggagaag gctggagaga tggtgcagag gttgagtgca 6360
   ctggctgctt ttacagagga cctgggttca attcctggcc tcacatggtg tctcacaaac 6420
   atctgtaact cagtttcagg gaatctgatt ctggtttctg gcctctttgg gatccaggga 6480
   tgcatgcagt acacagacat acacgcaggc aaaacatcca tacatgttca aaacaaaaca 6540
   aagcaactat gccaaaagag cataaaagca agtaaaatgt ctggctgatt ttaaattaag 6600
   acaattaatt taaaattagt taagacaatt aatttaaagt gctgaaaatt aattgttaaa 6660
   aatgatattt taaaaattaa tttccttttt tttttttaat tgtaaaaact acatctgtgt 6720
   atttcctggg aggggcagga cacttgccct ggcacttgtg gaagctcgag atgatgtgca 6780
   ggaattcgtt ctcttctccc accgtgtggg tcccagggat gcagtgcaga ttcatcagca 6840
   tcctggcagg cacctttatg tgctgagcca cctcaccacc catcctgtgt gttgggatca 6900
   tcctgtttct cttcttggtt gtcactaatt gtatgttggt actggcagtc tattcaggtg 6960
   ttttactctc tgacctaagt tcccattcag gctgggaatg taactcacta ataggataca 7020
   gcagaccctg gattccggct gtgtgaagga catttccctt cataatatcc tctgcagtgt 7080
   tggaataggc agcatgctta cttaatagac gatcacaaat tcaggacagt tgagattaag 7140
   atgagagtag gacacagctt tcaaacctac gtgtcctcag ggctcgagtg cagagtggtg 7200
   cagctatggt atctatgtgt gttttcacct ttttttgttt tgttttgttt tttgtttttt 7260
   gagacagggt ttctctgtgt agccctggct gtcctggaac ttactctgta gaccaggctg 7320
   gcctcaaact cagaaattcg cctgcctctg cctcccgagt gcttgggttt tcacttttaa 7380
   gtatggtcaa aagaatgtat cagtggatcc actctgaccc atggctttta ggcagagttg 7440
   tacctttcta gcttgttcat ggatctccct gccatattca ctctcaaaaa taaactctca 7500
   ggaaaagcta tgggaatttg gactggagat ctagctcaga ggtagaacac ttgcatgcgc 7560
   gcgcgcgcgc gcgcgcacac acacacacac acacacacac taaagccctg taggcaccag 7620
   atgacataaa attgactttc ataacagtgt ctatagagta agtaccagca tcattgctat 7680
   tacaaaggtc tgcaagctct tttatttggg ggcatatttt tctttttctt ccttttcttt 7740
   tttataattg atcagtcaga tttatatatc aataaattct cagttcatga gatatccaca 7800
   caatatgttc agagccgatg ataatgatac ggggtgggga gagtgcttat ttttcggaat 7860
   gttttctctt cgttgacaat tcagggacta gtgatatttc tacataggta gcttgattgg 7920
   agttttgtgg tttgtttttg tttttttttc agaaagacca ataggcacgg tgttctacag 7980
   caaagaatgg ttagactact ggtattgttc ccatttgttt ttacttaatt cctgagccaa 8040
   gtggccaaac agtaggtttc tgaaacaaaa tccatcaaac taattcctgc ctttgttgtc 8100
   agatacttgg ataagagaac cctcttaaaa taccaaacag cgacatccta tgccttaaat 8160
   tttgtgtaca aataatttct agttctgaat gtggtacact tgtgacccat ctcaatggat 8220
   gctgctttat ttagaatcca cctagtactg tttgacaagg ggtagaaaag aatgtgactc 8280
   ttcaccgttt ttctatttaa aatcatcaga atcatgtgtg atagtcactc agtccagtct 8340
   ttctgcctta acttaggtta ctagtggcct tgtagtacct ggcacagtca tcatctaaca 8400
   aaagacatgg cacccttaca agatctataa acattctacc caccaggatc cacagagaag 8460
   cacgggatta gctggcaaca cctcagcctc tggtgttgaa ggtgggcact tcatttggat 8520
   acttctcatg ccaccatctt tgggaaggag gatgagtcag aggtcatctt ttgaaaaatc 8580
   tggtcctgtt ccaatcattg tcttggaaat acatttccat cacatatcaa aatagtagct 8640
   atttcaccgg gatctttatt tcaagaaagc ctgtaagctc atttctgtgc tttcagcact 8700
   aaattcatcc cctcaccctg aacatctcca caagcaggag caggagcctc gctcagggca 8760
   gcttctcact ccaggcaagc aggttcctgc tgatactgca tgagggcaga gttacagtgt 8820
   cagctgtttt cctggctgtg taaaggatgg aggggaatat gccaaagcaa aaggcaattc 8880
   ttagcacaag atgcaccaca tggagttcta cagaaactgg agaaaagaca ttacaatttt 8940
   ctagggggcg gaacaaccca caggaaccca ccgacttgct ttcctttacc ctaagtggga 9000
   ttggagcaaa taaactcact caggtattta gtgtctgttc aaatagcttc aggtgtttag 9060
   cagaggaggt catgttaaat ataaattaca tgtttatctg agtccacaat ctgagtgcag 9120
   ccctagtgtg tcctgaagta atagtcctag agaaagaaca gtgggtgggg tgtgggaagg 9180
   acttttagat gccctgtacc atactgagac aagaaagggg tggagcaaat agacagtatt 9240
   cttccatctt taaccagttc cagacagcaa agaggaatag taggccagga agatgacttg 9300
   gtggctaaaa gcacttgcag ggtaagcagg acaacctagg tttggattca gagaacccac 9360
   gtaaaagcca aatgcagttc gcgcacattt gtaatcccaa cactcctgta gtcctgaagc 9420
   ccattgacca gctagactag agtgcacact gcagcagcag agacaacaga gaccctgcct 9480
   taacaggcag gaagtgaaaa taccctctga ctgggttctg gttcccgaca tgcacgatat 9540
   ggtatgcatc ttagtttctt ctcttgcttc aaagaaacac catgactagg gcaacatatt 9600
   aaaaacagca ttttattgaa ggcttgttta cagcttcaga aggttagtct atggccatca 9660
   tggtggagaa catgacatta ggcaggcagg catggcacta ggacagtagc ttagaactta 9720
   catgttatcc acagcatcag acaggatagc aagactgggt cttgtatggg cttttgaaac 9780
   ctcaaagccc atcctccaat gcgtgacaca cctcctgcaa taaggctgta ccactaaccc 9840
   ttcctaaaca gatgaccaac taggaaccaa acacacattt tgaggcctat ggggattatc 9900
   ctcattcaaa cccccacagt atgtttgcct gtacagacag acagatacat acagacatac 9960
   cccaaaacca aacctacact actaccaaac aaaacaaaac aaaacaaaat tggaaggaaa 10020
   atatgtttat cttttcctct taggttattc ctttgaaaga caatgtgttt ccataaaaac 10080
   aagaatcggg tgctgaagag atggtgatgt gattaaaagc acatattata ctgctctttc 10140
   aaaagatc 10148

<210> 6
<211> 20248
<212> DNA
<213> Artificial

<220>
<223> vector

<400> 6
acggaagatc acttcgcaga ataaataaat cctggtgtcc ctgttgatac cgggaagccc 60

tgggccaact tttggcgaaa atgagacgtt gatcggcacg taagaggttc caactttcac 120

cataatgaaa taagatcact accgggcgta ttttttgagt tatcgagatt ttcaggagct 180

aaggaagcta aaatggagaa aaaaatcact ggatatacca ccgttgatat atcccaatgg 240

catcgtaaag aacattttga ggcatttcag tcagttgctc aatgtaccta taaccagacc 300

gttcagctgg atattacggc ctttttaaag accgtaaaga aaaataagca caagttttat 360

ccggccttta ttcacattct tgcccgcctg atgaatgctc atccggaatt tcgtatggca 420

atgaaagacg gtgagctggt gatatgggat agtgttcacc cttgttacac cgttttccat 480

gagcaaactg aaacgttttc atcgctctgg agtgaatacc acgacgattt ccggcagttt 540

ctacacatat attcgcaaga tgtggcgtgt tacggtgaaa acctggccta tttccctaaa 600

gggtttattg agaatatgtt tttcgtctca gccaatccct gggtgagttt caccagtttt 660

gatttaaacg tggccaatat ggacaacttc ttcgcccccg ttttcaccat gggcaaatat 720

tatacgcaag gcgacaaggt gctgatgccg ctggcgattc aggttcatca tgccgtctgt 780

gatggcttcc atgtcggcag aatgcttaat gaattacaac agtactgcga tgagtggcag 840

ggcggggcgt aattttttta aggcagttat tggtgccctt aaacgcctgg tgctacgcct 900

gaataagtga taataagcgg atgaatggca gaaattcgag cttggcccag tgccaagctc 960

caatacgcaa accgcctctc cccgcgcgtt ggccgattca ttaatgcagc tggcacgaca 1020

ggtttcccga ctggaaagcg ggcagtgagc gcaacgcaat taatgtgagt tagctcactc 1080

attaggcacc ccaggcttta cactttatgc ttccggctcg tatgttgtgt ggaattgtga 1140

gcggataaca atttcacaca ggaaacagct atgaccatga ttacgccaag cttgcatgcc 1200

cctcgaggcc gctctagggc cgcatctagc tagagtcgat cgaccagctt ctgatggaat 1260

tagaacttgg caaaacaata ctgagaatga agtgtatgtg gaacagaggc tgctgatctc 1320

gttcttcagg ctatgaaact gacacatttg gaaaccacag tacttagaac cacaaagtgg 1380

gaatcaagag aaaaacaatg atcccacgag agatctatag atctatagat catgagtggg 1440

aggaatgagc tggcccttaa tttggttttg cttgtttaaa ttatgatatc caactatgaa 1500

acattatcat aaagcaatag taaagagcct tcagtaaaga gcaggcattt atctaatccc 1560

accccacccc cacccccgta gctccaatcc ttccattcaa aatgtaggta ctctgttctc 1620

acccttctta acaaagtatg acaggaaaaa cttccatttt agtggacatc tttattgtta 1680

atagatcatc aatttctgca tcctcgactc tagtggatct gcattccacc actgctccca 1740

ttcatcagtt ccataggttg gaatctaaaa tacacaaaca attaggaatc agtagtttaa 1800

cacattatac acttaaaaat tttatattta ccttagagct ttaaatctct gtaggtagtt 1860

tgtccaatta tgtcacacca cagaagtaag gttccttcac aaagagatcg cctgacacga 1920

tttcctgcac aggcttgagc catatactca tacatcgcat cttggccacg ttttccacgg 1980

gtttcaaaat taatctcaag ttctacgctt aacgctttcg cctgttccca gttattaata 2040

tattcaacgc tagaactccc ctcagcgaag ggaaggctga gcactacacg cgaagcacca 2100

tcaccgaacc ttttgataaa ctcttccgtt ccgacttgct ccatcaacgg ttcagtgaga 2160

cttaaaccta actctttctt aatagtttcg gcattatcca cttttagtgc gagaaccttc 2220

gtcagtcctg gatacgtcac tttgaccacg cctccagctt ttccagagag cgggttttca 2280

ttatctacag agtatcccgc agcgtcgtat ttattgtcgg tactataaaa ccctttccaa 2340

tcatcgtcat aatttccttg tgtaccagat tttggctttt gtataccttt ttgaatggaa 2400

tctacataac caggtttagt cccgtggtac gaagaaaagt tttccatcac aaaagattta 2460
gaagaatcaa caacatcatc aggatccatg gcgaggacct gcagggtcgc tcggtgttcg 2520
aggccacacg cgtcacctta atatgcgaag tggacctggg accgcgccgc cccgactgca 2580
tctgcgtgtt cgaattcgcc aatgacaaga cgctgggcgg ggtttgctcg acattgggtg 2640
gaaacattcc aggcctgggt ggagaggctt tttgcttcct cttgcaaaac cacactgctc 2700
gacattgggt ggaaacattc caggcctggg tggagaggct ttttgcttcc tcttgaaaac 2760
cacactgctc gactagaact aattcttatc gtatttcatt tttgattaaa tttagatttt 2820
gtgtgtgtgt atgtgtgtgt gtgtagagga gtatatatgc tcatctgagt gcaggtgcca 2880
ttgaagtcca gaagatggtg taggatacct tggatctgga gtcataggca gctatgagcc 2940
tcccagtgta aacactgatt taaaaaaaaa aatcaacatt tttatatagc caaatgaact 3000
gatgacaaag ctgggtaaaa tggtatatgc ctgtattccc agtggcaggc agaatgggag 3060
gtctcaaatt ctaagccaat ctatataatg aaacgctagc acaaaacaaa gctctgaaaa 3120
atgttttctt gatagtatca ttgaatttat tctgaccttt aaagtgttct ttttgaattg 3180
gcagtaaaag attttatgtt gatacatggt aatataaatt ttttaaaaac aatatatgca 3240
tgtagtcttg tattcattga agatacttta gaataatatt tagagaaata tatattagta 3300
acccagtatt taataagcat tagagatttt ttccccaaac atggaaaaca ttatttaatt 3360
tgacaattat aacaggaaat gttcttttgg gtagtctcaa gttctttaga ataacaagac 3420
ttaaaaatac cagttctgaa ctgaacagaa aacataaaag gaggaaatga gaatattaaa 3480
gcttacctgt tctacaaagc aagatttcgt gtcataaaga tgtcatttga tttatgacac 3540
aactcagaag ccagtgtagt tataagagaa aagataaaaa gaatgaccaa aagtagagaa 3600
gtgggactgg ggagatgttt cagcaatgaa gaatattttc tgatcttgca gagagaatat 3660
gctctgttaa gggactgaat caacacagta gaagcagagt caggagctat gattcaaagt 3720
cactaatata gcttcccaga gtcctcaaca acctgctctt taaaagacga catgccatct 3780
tcatttatct caaattacac caccgcattc ctgagataaa ttagtcatgt tgccattata 3840

aaacatatgc cacataacta gacttcctct ctggatccta tgaatgtgta acatactcat 3900

ctaaccccaa taaaatatga aatgtgctta gtaaataaaa tgtaacatcc cttatatcag 3960

tattctgaag caacataaga aatcaagtgg tgggctggag agatggctca gtgattaaga 4020

gcaccggctg ctcttccaaa ggtcgtgagt tcaaatccca gcaaccacat ggtggctcac 4080

aaccatccat aatgagatct gacgacctct tctggtgcat ctgaagacag ctacagtgta 4140

cttagataca ataataaata aatctttaaa aaaaaaaaaa gaaatcaagt ggtatccatt 4200

ctcaagtcta tagcaaaaca tatctacatg gtagactttt ttacttttta tcagataatt 4260

ttatatgtca taaaattcta catggcaggt tttaggaaga atttttaaaa atgtctttag 4320

ttatctatcc ataagtaacg tgatggatgt tttagttttt ggtatttctt atcacctgtt 4380

ttttcagagg tgacaaatgt gatatcaaga acttgctggg gaccaaaagg agtagagtca 4440

ctgaccttgt ccataaatgt acacaggtag tgattataat tatgctcttc tccttcaccc 4500

catcagtaaa ctgaattggc ctgaaactct ttgtatactt tctgtctttg ttttctaagt 4560

gttaaatcgt tgtttttatg gtactcatta aagagaaaag gaaggataac tgtaacttga 4620

agtcgctgtc ttttggattg gcttatgtga agatcacttt ttagtagtta gcgcctatcg 4680

cactgtggtg ttaggatcaa gcttatcgat accgtcgatc gacggtatcg ataagcttga 4740

tatcgaattc ctgcagccgg ccgcacgtct aagaaaccat tattatcatg acattaacct 4800

ataaaaatag gcgtatcacg aggccctttc gtcttcaaga attccgatca tattcaataa 4860

cccttaatat aacttcgtat aatgtatgct atacgaagtt attaggtctg aagaggagtt 4920

tacgtccagc caagcttagg atcgatccga acaaacgacc caacacccgt gcgttttatt 4980

ctgtcttttt attgccgatc ccctcagaag aactcgtcaa gaaggcgata gaaggcgatg 5040

cgctgcgaat cgggagcggc gataccgtaa agcacgagga agcggtcagc ccattcgccg 5100

ccaagctctt cagcaatatc acgggtagcc aacgctatgt cctgatagcg gtccgccaca 5160

cccagccggc cacagtcgat gaatccagaa aagcggccat tttccaccat gatattcggc 5220

aagcaggcat cgccatgggt cacgacgaga tcctcgccgt cgggcatgcg cgccttgagc 5280

ctggcgaaca gttcggctgg cgcgagcccc tgatgctctt cgtccagatc atcctgatcg 5340

acaagaccgg cttccatccg agtacgtgct cgctcgatgc gatgtttcgc ttggtggtcg 5400

aatgggcagg tagccggatc aagcgtatgc agccgccgca ttgcatcagc catgatggat 5460

actttctcgg caggagcaag gtgagatgac aggagatcct gccccggcac ttcgcccaat 5520

agcagccagt cccttcccgc ttcagtgaca acgtcgagca cagctgcgca aggaacgccc 5580

gtcgtggcca gccacgatag ccgcgctgcc tcgtcctgca gttcattcag ggcaccggac 5640

aggtcggtct tgacaaaaag aaccgggcgc ccctgcgctg acagccggaa cacggcggca 5700

tcagagcagc cgattgtctg ttgtgcccag tcatagccga atagcctctc cacccaagcg 5760

gccggagaac ctgcgtgcaa tccatcttgt tcaatggccg atcccatatt ggctgcaggt 5820

cgaaaggccc ggagatgagg aagaggagac agcgcggcag acgtgcgctt ttgaagcgtg 5880

cagaatgccg ggcctccgga ggaccttcgg gcgcccgccc cgcccctgag cccgcccctg 5940

agcccgcccc cggacccacc ccttcccagc ctctgagccc agaaagcgaa ggagccaaag 6000

ctgctattgg ccgctgcccc aaaggcctac ccgcttccat tgctcagcgg tgctgtccat 6060

ctgcacgaga ctagtgagac gtgctacttc catttgtcac gtcctgcacg acgcgagctg 6120

cggggcgggg gggaacttcc tgactagggg aggagtagaa ggtggcgcga aggggccacc 6180

aaagaacgga gccggttggc gcctaccggt ggatgtggaa tgtgtgcgag gccagaggcc 6240

acttgtgtag cgccaagtgc ccagcggggc tgctaaagcg catgctccag actgccttgg 6300

gaaaagcgcc tcccctaccc ggtagaattg acctgcaggg gccctcgagg ggtcgacggt 6360

atcgataagc ttgatccttg tgagagtact tagttgagaa gcggtaaggt aggtgcctat 6420

caaggactct gaaccatctc agggactcag catgtaggag cattcatctc tttctctgtt 6480

tctctctaat ttctaaccat cttttaaggc caagttcttc tccaaacctt tattgagcta 6540

ttcacctgct caaattcctg atgatatctg ttctttgatc tttctttact cttcctttga 6600

tactggtttc acatccataa caggagtttt aaattccata tgagcagaaa ttacttccca 6660

ttatctttgg atcttagtat gtaatgcagt ggcacataag agaggctaaa gtattttctg 6720

actctttggc ttaggtacag cttatacaga attttttctt ttcttaaaca cagaaattta 6780

aagacgacta cagtgaactt gcttgttctg gcacagttgc caagtattgg tctctctttg 6840

gttacttgaa gattatctgg tctgagagaa gagggtttta aaagggccct aaagaaaact 6900

agtagaacgt aagttgttca acagcctcgc tgttcattca gttcactctc ttacaggtac 6960

tgcagaagga gaggctgggt gcgttccaca ctcatcatgt ctgtgccaca aacaagtaca 7020

tctaaaggga aagtcatgct taaagagtac agtggacgca agattgaagt agagcacatt 7080

tttaagtgga taactgccca tgcagcttct cggatcaaaa ctatatataa tgtggagcat 7140

ttgaaggaag aatggaataa aagtgatcaa tactgggtaa aaatatacct atttgcaaac 7200

cttgaccaac cgccagcttt cttctctgca ttaagtataa aatttactgg aagagttgag 7260

tttatttttg ttaatgtgga aaattggaac aacaagagtt atatgacaga tattggtatt 7320

tataacatgc catcatacat acttagaact cctgaaggaa tttatagata cggaaaccac 7380

acaggtgaat ttatatccct tcaggccatg gattcatttc tgcgctcatt acaacctgaa 7440

gtaaatgatc tgtttgtttt gagtttggtt ctagttaatc ttatggcttg gatggactta 7500

tttattacac aaggagcaac catcaagcga tttgtggttc tcataagcac tttaggacaa 7560

tacaattccc tattaattat ttcttggcta cctgtgttgg gctttctaca gctcccttac 7620

ttagatagct tttatgaata tagtttaaga ttgctgcgat attctaatac aaccacactg 7680

gcttcgtggg taagggcaga ctggatgttt tactcttcac acccagccct gtttctcagt 7740

acataccttg gacatggttt gctaattgat tactttgaga agaagagacg tcgcagcaac 7800

aatgatgaag ttaatgcgaa taatttagaa tggttatcaa gtctgtggga ctggtacacc 7860

agctacctct tccacccgat tgcttctttt cagaactttc ctgtagactc tgattgggac 7920

gaagaccctg acttattctt ggaacggtta gctttccctg acctttggct tcaccctctg 7980

ataccaactg attatattaa aaacttacca atgtggcgat ttaaatgtct tggggttcag 8040

tctgaagaag aaatgtcaga gagttctcaa gacactgaaa atgactcgga tagtgacaac 8100

atggacactt ttagtagtag taaggatata tttgaagata aacaaagcgt tgttcacagt 8160

tctccaggaa gaacaagtca ctgtgatact gaggcttgtt catgtgccaa taaatgtgag 8220

agcagcccat gtgaaaggaa gaggaggtcg tatggatcac ataatactga tgaagatatg 8280

gaaccggact ggttaacttg gcctgcgggt acgctgcact gtactgaatg tgttgtttgc 8340

cttgagaatt ttgaaaatgg atgtttgcta atggggttgc cttgtggtca cgtgtttcac 8400

cagaattgca ttgttatgtg gttggctggg ggccgacact gttgccctgt ttgtcgttgg 8460

ccttcatata agaaaaagca gccatatgca caacaacagc cgttgtcaaa tgatgttcca 8520

tcttaaccat gtgcaatttg tccttaataa gccttgagta tcttacagct tgccttttta 8580

atgttagtca caatgttttt gtggtttgaa gtttagttta atgttagtgc agtgacagga 8640

aatacacatt atgctgatgt tgatgacaga atttatttgg ttgccttgtg tgtcaattga 8700

atgcatacta aactgtaaaa aaaatttatt tacagcattg aaaattcaga agttaatggt 8760

tttttgtaag cacaaaagaa gtatggtaga aattaatctt aacaagactt tatgaggcag 8820

gatcaaatcc tagtgggcct gagcaggttt cttaccctaa gtgttttctc cctttttaca 8880

atctctgtcc agcacctctt ggttaaataa tgtatgctct gagacatgaa attaaaacag 8940

atcatcgaat tcctgcagcc ggaaccctta atataacttc gtataatgta tgctatacga 9000

agttattagg tccctcgaag aggttcacta gtactgggta cccgatctat aaaataaatt 9060

attttaaaag cagaaggttg taggtttctg atcttaagct gtggtaaggt gactggtttt 9120

tgtaggtgtt ttgctggatt actgatttct cgggtaattg caacgtgatt ttgagatgga 9180

gggagaagaa atggaatcct tttgctgaca ctggctatga gtaatggtgg tctacctcac 9240

agcctaatat cttggatagg aaagaatttt atagggggct ttgaaaaatc agtacttagc 9300

atattcatct aagtaaaaac aactataact ttttagtcac acaaggatta ccttgtatgt 9360

ttaactgact ttttattttg taagttttta gtggtagagg aaagccgagc atatagatag 9420

tagaaaaaat atctttctct ttcgtgtacc atatttgtat tttattacat ttactttagc 9480

accttttatt acaattttaa tacttcttct ttttccctct cagtcagctg aaagtaaact 9540

tgaatcatga tgctttgccc gtaaacagtc accacatttt ctgtgaatgt cccccatacc 9600

ctctccccca ctcccctacc cacccattcc cactttttgg ccctggcgtt cccctgtact 9660

ggggcatata aggtttgcgt gtccaatggg cctctctttc cagtgatggc tgactaggcc 9720

atcttttgat acatatgcag ctagagacac gagctccggg gtactggtta gttcatattg 9780

ttgttccatc tataggattg cagaccccat tagctccttg ggtactttct ctagctcctc 9840

cattgggggc cctgtgatcc atccaatagc tgactgtgag catccacttc tgtgtttgct 9900

aggcccctgc atagtctcac aagagacagc tatatcaggg tcctttcagc aaacgcttgc 9960

tagtgtatgc aatggtgtca tcgtttggag gctaattatg ggatggctcc ctggatatgg 10020

cagtctctag atgatccatc cttttgtctc agctccaaac tttgtctctg ttactccttc 10080

catgggtgat tgtttccaat tctaagaagg ggcaaagtgt ccacactttg gtctttgttc 10140

ttcttcagtt tcgtgtgttt tacaaattgt ctcttatatt tcgggtatac taagtttctg 10200

ggctaatatc cacttatcag tgagtacata tcatttgagt tcttttgtga ttgtgttacc 10260

tcactcagga tgatgccctc caggtccaac catttgccta ggaatttcat aaattcattc 10320

tttttaatag ctgagtagtg aacccttgac atttaatacc taatatttgt tccttgtccc 10380

ataaaagtct ttatatctct gtctttgttt tgttgactta tctgttgggt ttagtttccc 10440

tttcttccat cttcccttag ttcctgtaga gaaactgctg ttcatgtgtg tgtgtgtgtt 10500

gtgctaaggc ttgaattcag gacctcagca taataggcaa gtaccccaag ctacacccca 10560

gccctctcta aattttttaa ttttgagata gtctcactag attgcctgag ttggtcttga 10620

actcattata gcttaggtaa gccttgagtt ctcaatcttc ctgcatcagc ctcttagatt 10680

acatgcctga gttgcaagcc tgatgcctgt tctggtacta atatttttaa cagtacaaat 10740

tggcttatat agtgttcctt gttttctttc aagattaaat ttgagtgctg ctcatataca 10800

tcgtatcagg atgcatgtag tgtcagttct gttgttggtg attcgaaatg tgtttaatat 10860

ctgtcaagtc tttcctttgt gagatacctt ttccctctgt aatttaagaa gcaacctgtg 10920

attctttgtg atcttttccc tcatctcagt ggttttaaca gccagtgatg attctttgac 10980

ttggtcatca agttagaata tggtcatttt caaatttctg cttaattgca catttattag 11040

ctggctgtgt gtgtgagaga gaggcgagga gagctttcta tttttctccc atgttttgaa 11100

tatcactgga cttagatgtt tatcattatt attattatta ttattattat tattattatt 11160

attacccagt gtattaaaac tttaggtgct agagagaatg ctcgttggtt aagagcactg 11220

gctactctta caggagacct gggtttgatt cccagcatcc atatgatagc caactagcca 11280

ccatcttatt tgtaactaca gtttcaggag gtctaataca tttcttgcct ctgtaggcag 11340

tgaacacaca aggtacacag gcaaaacacc catgtaagta agtaagtaag taagtaagaa 11400

agacagtcag tcagtcagtc agtcccaaag cactcataaa aattattaaa ttttaaaaaa 11460

tgcttatttt tggtactcag tgtcatactt agctagtgga aacctcttta cactagcctt 11520

atgtcttgtt actaatgtct taatcctgaa gcacttgttt gctttttgac cctggtttct 11580

tgtcatcttt ttttcagagt acccaagttt catgatggac acctctaaga tccttagtta 11640

gctgcactcc aaaatggaaa tgggaaaaac aaaatataaa ttatttccag gatactaaca 11700

atttttcttt gcatgataaa ttgtgcctta ctttcaaata ctaatagaaa ataggtgctt 11760

tttcactttt agtgggatta tgtcataata aatccatcat aaattgaaag tttcacaagt 11820

aaaacatgca tttacaagtc taaagtccca actttacgta gaaaaatgtc ggttatcctt 11880

caaggtcctc tgactggcag ttccagcttg atacctctat ccagaatact gtactttcct 11940

aggctgaaca gtgaaattct agtcagaatt tcaactaact acctctaaag agacagactt 12000

ctttgtcatc atcctaaaaa ttgagaaacc atcaaacatc agcagaatgc ttgtctaaat 12060

agagaattca taaagccctg aattggtcct cagcaccata taaactgaca tggtggtgca 12120

tacctgtcgt cccactcaga ggtggaggat caagggtcca gttcatgttc aactacatag 12180

agagttcaaa actaacctga gagatattag accttgtctt aaataggaaa acacctagta 12240

gaaaggacta gcagctatgg attgtatttc ataaatttga gaacttaaaa taactgcagc 12300

cattgctgca tatacttctg aacaagcgct gaaatccttt ccttgtaaat ggagtcccaa 12360

gatttctcac ctcttctttt gtccatgctt tcagcagatg gttctcaaat ttacctgttc 12420

ttcatgggct ctttccacag caccagtttt atacacagat ttctactaga ctcgcttgaa 12480

gtacttcgga tcgaacttcg gtcttttggg gcatactagg caagcactct cccatcctca 12540

gcctttgatt tttgtagctt gggatggcct tgaactcata atcatcctga attcaattct 12600

gccatgccct ggctctgggt tcttttaatt aactacagtg ctctgttgtc tttcttcatc 12660

cccagtcttc cttttaaggt cttaatggag ataggtggag ttacaaaatg tacatgaaag 12720

tgtagaaatg tttagacaaa atttctctag ctttccagag cacatcacac attggatatc 12780

attatcctaa acagaccttt tgaatctttt ccattcataa tccctttcat tcaagaaggt 12840

ggctttgggg catgtaggta tataaaatag ctacacatat cagacattta ctggcaataa 12900

aattgtaaat atatttaaaa gcctctgcta tccctacagc tttgctgttc actaaagagt 12960

aagacagttt ttcatactag taagataagt gtgcttattt ttacataaag aattaaatct 13020

tggggttgga gagatggctc aacagttaag agcactgact attcttccag ggctcttaag 13080

ttcaattctc agcaaccaca tagtggctca caaccatctg taatgggatc caatgcccgg 13140

atctgttgtg tctgaagaca gctacagtgt actcatgcac ataaataaat ctttaaaaaa 13200

aaagaattaa atcttagcca agtgtagtgg tacacacctc taatccaagg gaggcagagg 13260

caggtggatc ttcctgagtt tgaggccaac ctggtctata ataaagtccc agaacagaca 13320

gggctgttac ataaagaaac cctgccttga aaaagtcaaa acaaccccaa aataatcttg 13380

tctgaatatt ttatgttagc ctagaagccc agcacatctt tgacattatt tagaactgat 13440

caacattttt atttttatga ttgtcagtgc agaagatgca actttgtata aatctgtaat 13500

ataaaatgaa aaaaaagcag aaatatgttt agagccattt cagaaattgt tgaaaattct 13560

gcctaatccc atgtcaaaat tgatttaaca gtttttaagg aaactgagtc agcaactggc 13620

agtttttaaa gaatctgata caatccagtc taaacagttg ctagctatac tagtatactg 13680

atatggtagt tacatgctaa gaaatgacaa taaagaaaaa ttttaagctt ttgctttctg 13740

tgattaaacc attatgtttc tgtaagagca gaaaactgta tatacactta aaatactgca 13800

gttcaccacg tgatagcgtt gagtctgagc caagatgttt caggcagttt gtgttggcac 13860

tgtgtggcat ccctacacgt gaagtgcagc atgtgtgttg tatgttcaga gacaaggctg 13920

tggtgaagcc aggctgtgaa acacaggtga atgttaccag aaacacctta gattcatcat 13980

ctgtctgatt ctgtattacc tttgttcttt tgcaaattca ggaagttttt actcttaaaa 14040

catttatcca gacccccaaa tttagttact ggaccaagtt gaggactaga caacaaagaa 14100

atcagcattg gccatcaatg gtccctctca tttctggctt actatttagg ctcattttca 14160

tttcacattc ttttttttta agacttttat ttatttatta tatgtgagta cactgtagct 14220

gtcttcagac actctggatg agggtgtcag atcttgttac agatggttgt gagccaccat 14280

gtggttgctg ggatttgaac tcaggacctt tggaagagca gtcgatgttc ttaaccactg 14340

agccatctca ccagccctca tttcacattc ttaccacccg tgttattttt cctccggtgg 14400

gtgctttgtt gtcatcccct ctacctggtt gtattgttcc tttttctact ctttctgttg 14460

gcttggttga attttctccc taagttctgc gccttcccat cttctgcttc cttactagac 14520

ttaaaagggg gcaaagatac ttacaggtag tgacagtcag tgaaggacac cttattcaag 14580

tgattcaact aatgtcatca acagctgagt ggagagatgt gtgctctatg tttcaggtac 14640

tttggacatt cctgatagat gcattgaact aaacattgca tgtgcctttt tagatccttt 14700

atggtagggc ctgtgtgtta ttaatctagc tacccacaat gcatggtcca aaatggacaa 14760

aatacttttt tcagtgttat gtgtgcacaa aaattcctca gtgaaaaaac aaaattactt 14820

caaaatggtt agagggactg ccagggtttc gggtcttcat acacgtggct catcccttaa 14880

ttgttaaatc tgctcttagc agattttccc tgaggtagag aactgaagga aatactgaag 14940

attgatagat tttatttcaa cttgttttta aagaaaagtt ctgtttcttt gaacagtttc 15000

ttgtatttaa ggaaccccaa gcatgtggtc tttgcaggtg caggattccc tggataatat 15060

gacacataat ttgttaggag acttttgaaa cctaaattat tttgtgtgta aaatctcttc 15120

attctgtaaa gttgataagc tccctgaggg gtgaaatatt atgtagaatt gcctcctctc 15180

agtatgtaag atcctatata gatacagcaa ataattgctg gcttgaatct taagaacata 15240

ttttagtttt acctctctcc catctcatgt aggtaaggaa ggtaaaagat gctatttact 15300

ttttaggtta cttgggagcc tggcaagtta ttcttgtaaa atctttctct tttctcacct 15360

gtgttttcat tttattattc cttaaaactc agtcaaagga gaaggctgga gagatggtgc 15420

agaggttgag tgcactggct gcttttacag aggacctggg ttcaattcct ggcctcacat 15480

ggtgtctcac aaacatctgt aactcagttt cagggaatct gattctggtt tctggcctct 15540

ttgggatcca gggatgcatg cagtacacag acatacacgc aggcaaaaca tccatacatg 15600

ttcaaaacaa aacaaagcaa ctatgccaaa agagcataaa agcaagtaaa atgtctggct 15660

gattttaaat taagacaatt aatttaaaat tagttaagac aattaattta aagtgctgaa 15720

aattaattgt taaaaatgat attttaaaaa ttaatttcct tttttttttt taattgtaaa 15780

aactacatct gtgtatttcc tgggaggggc aggacacttg ccctggcact tgtggaagct 15840

cgagatgatg tgcaggaatt cgttctcttc tcccaccgtg tgggtcccag ggatgcagtg 15900

cagattcatc agcatcctgg caggcacctt tatgtgctga gccacctcac cacccatcct 15960

gtgtgttggg atcatcctgt ttctcttctt ggttgtcact aattgtatgt tggtactggc 16020

agtctattca ggtgttttac tctctgacct aagttcccat tcaggctggg aatgtaactc 16080

actaatagga tacagcagac cctggattcc ggctgtgtga aggacatttc ccttcataat 16140

atcctctgca gtgttggaat aggcagcatg cttacttaat agacgatcac aaattcagga 16200

cagttgagat taagatgaga gtaggacaca gctttcaaac ctacgtgtcc tcagggctcg 16260

agtgcagagt ggtgcagcta tggtatctat gtgtgttttc accttttttt gttttgtttt 16320

gttttttgtt ttttgagaca gggtttctct gtgtagccct ggctgtcctg gaacttactc 16380

tgtagaccag gctggcctca aactcagaaa ttcgcctgcc tctgcctccc gagtgcttgg 16440

gttttcactt ttaagtatgg tcaaaagaat gtatcagtgg atccactctg acccatggct 16500

tttaggcaga gttgtacctt tctagcttgt tcatggatct ccctgccata ttcactctca 16560

aaaataaact ctcaggaaaa gctatgggaa tttggactgg agatctagct cagaggtaga 16620

acacttgcat gcgcgcgcgc gcgcgcgcgc acacacacac acacacacac acactaaagc 16680

cctgtaggca ccagatgaca taaaattgac tttcataaca gtgtctatag agtaagtacc 16740

agcatcattg ctattacaaa ggtctgcaag ctcttttatt tgggggcata tttttctttt 16800

tcttcctttt cttttttata attgatcagt cagatttata tatcaataaa ttctcagttc 16860

atgagatatc cacacaatat gttcagagcc gatgataatg atacggggtg gggagagtgc 16920

ttatttttcg gaatgttttc tcttcgttga caattcaggg actagtgata tttctacata 16980

ggtagcttga ttggagtttt gtggtttgtt tttgtttttt tttcagaaag accaataggc 17040

acggtgttct acagcaaaga atggttagac tactggtatt gttcccattt gtttttactt 17100

aattcctgag ccaagtggcc aaacagtagg tttctgaaac aaaatccatc aaactaattc 17160

ctgcctttgt tgtcagatac ttggataaga gaaccctctt aaaataccaa acagcgacat 17220

cctatgcctt aaattttgtg tacaaataat ttctagttct gaatgtggta cacttgtgac 17280

ccatctcaat ggatgctgct ttatttagaa tccacctagt actgtttgac aaggggtaga 17340

aaagaatgtg actcttcacc gtttttctat ttaaaatcat cagaatcatg tgtgatagtc 17400

actcagtcca gtctttctgc cttaacttag gttactagtg gccttgtagt acctggcaca 17460

gtcatcatct aacaaaagac atggcaccct tacaagatct ataaacattc tacccaccag 17520

gatccacaga gaagcacggg attagctggc aacacctcag cctctggtgt tgaaggtggg 17580

cacttcattt ggatacttct catgccacca tctttgggaa ggaggatgag tcagaggtca 17640

tcttttgaaa aatctggtcc tgttccaatc attgtcttgg aaatacattt ccatcacata 17700

tcaaaatagt agctatttca ccgggatctt tatttcaaga aagcctgtaa gctcatttct 17760

gtgctttcag cactaaattc atcccctcac cctgaacatc tccacaagca ggagcaggag 17820

cctcgctcag ggcagcttct cactccaggc aagcaggttc ctgctgatac tgcatgaggg 17880

cagagttaca gtgtcagctg ttttcctggc tgtgtaaagg atggagggga atatgccaaa 17940

gcaaaaggca attcttagca caagatgcac cacatggagt tctacagaaa ctggagaaaa 18000

gacattacaa ttttctaggg ggcggaacaa cccacaggaa cccaccgact tgctttcctt 18060

taccctaagt gggattggag caaataaact cactcaggta tttagtgtct gttcaaatag 18120

cttcaggtgt ttagcagagg aggtcatgtt aaatataaat tacatgttta tctgagtcca 18180

caatctgagt gcagccctag tgtgtcctga agtaatagtc ctagagaaag aacagtgggt 18240

ggggtgtggg aaggactttt agatgccctg taccatactg agacaagaaa ggggtggagc 18300

aaatagacag tattcttcca tctttaacca gttccagaca gcaaagagga atagtaggcc 18360

aggaagatga cttggtggct aaaagcactt gcagggtaag caggacaacc taggtttgga 18420

ttcagagaac ccacgtaaaa gccaaatgca gttcgcgcac atttgtaatc ccaacactcc 18480

tgtagtcctg aagcccattg accagctaga ctagagtgca cactgcagca gcagagacaa 18540

cagagaccct gccttaacag gcaggaagtg aaaataccct ctgactgggt tctggttccc 18600

gacatgcacg atatggtatg catcttagtt tcttctcttg cttcaaagaa acaccatgac 18660

tagggcaaca tattaaaaac agcattttat tgaaggcttg tttacagctt cagaaggtta 18720

gtctatggcc atcatggtgg agaacatgac attaggcagg caggcatggc actaggacag 18780

tagcttagaa cttacatgtt atccacagca tcagacagga tagcaagact gggtcttgta 18840

tgggcttttg aaacctcaaa gcccatcctc caatgcgtga cacacctcct gcaataaggc 18900

tgtaccacta acccttccta aacagatgac caactaggaa ccaaacacac attttgaggc 18960

ctatggggat tatcctcatt caaaccccca cagtatgttt gcctgtacag acagacagat 19020

acatacagac ataccccaaa accaaaccta cactactacc aaacaaaaca aaacaaaaca 19080

aaattggaag gaaaatatgt ttatcttttc ctcttaggtt attcctttga aagacaatgt 19140

gtttccataa aaacaagaat cgggtgctga agagatggtg atgtgattaa aagcacatat 19200

tatactgctc tttcaaaaga tcgagtcgac cattgcggcc gccaccgcgg tggagctcga 19260

attcactggc cgtcgtttta caacgtcgtg actgggaaaa ccctggcgtt acccaactta 19320

atcgccttgc agcacatccc cctttcgcca gctggcgtaa tagcgaagag gcccgcaccg 19380

atcgcccttc ccaacagttg cgcagcctga atggcgaatg agcttcttcc gcttcctcgc 19440

tcactgactc gctgcgctcg gtcgttcggc tgcggcgagc ggtatcagct cactcaaagg 19500

cggtaatacg gttatccaca gaatcagggg ataacgcagg aaagaacatg tgagcaaaag 19560

gccagcaaaa ggccaggaac cgtaaaaagg ccgcgttgct ggcgtttttc cataggctcc 19620

gcccccctga cgagcatcac aaaaatcgac gctcaagtca gaggtggcga aacccgacag 19680

gactataaag ataccaggcg tttccccctg gaagctccct cgtgcgctct cctgttccga 19740

ccctgccgct taccggatac ctgtccgcct ttctcccttc gggaagcgtg gcgctttctc 19800

aatgctcacg ctgtaggtat ctcagttcgg tgtaggtcgt tcgctccaag ctgggctgtg 19860

tgcacgaacc ccccgttcag cccgaccgct gcgccttatc cggtaactat cgtcttgagt 19920

ccaacccggt aagacacgac ttatcgccac tggcagcagc cactggtaac aggattagca 19980

gagcgaggta tgtaggcggt gctacagagt tcttgaagtg gtggcctaac tacggctaca 20040

ctagaaggac agtatttggt atctgcgctc tgctgaagcc agttaccttc ggaaaaagag 20100

ttggtagctc ttgatccggc aaacaaacca ccgctggtag cggtggtttt tttgtttgca 20160

agcagcagat tacgcgcaga aaaaaaggat ctcaagaaga tcctttgatc ttttctacgg 20220

ggtctgacgc tcagtggaac tccgtcga 20248

<210> 7
<211> 3261
<212> DNA
<213> mouse

<400> 7
cggctacagc gggtccagtg ctctgggggc gacggccgcg gcctgcagag gcggcaggga 60

cggtggccgc ggttggcgcg cgcgtccgca cggggctggg caccgcgcgg ctacgcagct 120

gacaggaagc cgcccgggag agccgcgttg gggcctagcg ttatttgctt tttcctcttt 180

ttttcctccc ttcacgactg tcgtctcgcg ctcctccgca gcgggagccg ccgcgacgcc 240

ccctcgcggg ccgcggggcc tgataggcgc cgcccgcggg aacctggagc tgccgccagg 300

cctgggcggc cgccggggcc tgaagcctgg gcgttcggcg cggcgctgcg gcggtcactc 360

caacccgcgc tgggcgcgcc gggccccggg cctggcccag ccgagtaccg cgccctctgg 420

accactcagt ccggccccaa ccgctctccg ctggcccgca gtccctcgga gctgcctccc 480

ggtcacccca ggtgggctct tgccctcttc cagccgtccg cagctcgatg ggtggggtgc 540

gtttgaacgt ccccccttct ccctttctca gctcctgacc gcaaggccag agccggggct 600

ctcagtcgca cgcacgccgc gatccacctg cctccccgcg ggatggcccc gccggtgacc 660

gccgcgcgcc ctcgctgccc tcgcccaggc tgcgctctgt cgccccgcac tcgatcccct 720

tcccttgtcc acggcgtccc ggctcctggc gacgccaaga gggcgaagat gtggctgaag 780

ctgtttttct tgctcctgta tttcctggtc ctgttcgtcc tggccaggtt ttttgaggcc 840

attgtgtggt acgagactgg catctttgct actcagctgg tagatccggt ggcattgagc 900

ttcaagaagc tgaagaccat tctggagtgt cgagggctgg gctactccgg gctgccggag 960

aagaaagatg ttcgggtgct ggtggagaag tcaggtgact tgatggaagg tgaactctat 1020

tctgctctta aggaagaaga ggcatctgaa tctgtttcta gtaccaattt cagtggtgaa 1080

atgcatttct atgagcttgt ggaagacaca aaagatggca tctggctggt tcaggtcata 1140

gcaaatgaca gaagtccttt ggtgggtaaa atccactggg agaaaatggt gaaaaaagtg 1200

tcaagatttg gaatacggac aggcactttc aactgttcca gtgatcccag gtactgcaga 1260

aggagaggct gggtgcgttc cacactcatc atgtctgtgc cacaaacaag tacatctaaa 1320

gggaaagtca tgcttaaaga gtacagtgga cgcaagattg aagtagagca catttttaag 1380

tggataactg cccatgcagc ttctcggatc aaaactatat ataatgtgga gcatttgaag 1440

gaagaatgga ataaaagtga tcaatactgg gtaaaaatat acctatttgc aaaccttgac 1500

caaccgccag ctttcttctc tgcattaagt ataaaattta ctggaagagt tgagtttatt 1560

tttgttaatg tggaaaattg gaacaacaag agttatatga cagatattgg tatttataac 1620

atgccatcat acatacttag aactcctgaa ggaatttata gatacggaaa ccacacaggt 1680

gaatttatat cccttcaggc catggattca tttctgcgct cattacaacc tgaagtaaat 1740

gatctgtttg ttttgagttt ggttctagtt aatcttatgg cttggatgga cttatttatt 1800

acacaaggag caaccatcaa gcgatttgtg gttctcataa gcactttagg gacatacaat 1860

tccctattaa ttatttcttg gctacctgtg ttgggctttc tacagctccc ttacttagat 1920

agcttttatg aatatagttt aagattgctg cgatattcta atacaaccac actggcttcg 1980

tgggtaaggg cagactggat gttttactct tcacacccag ccctgtttct cagtacatac 2040

cttggacatg gtttgctaat tgattacttt gagaagaaga gacgtcgcag caacaatgat 2100

gaagttaatg cgaataattt agaatggtta tcaagtctgt gggactggta caccagctac 2160

ctcttccacc cgattgcttc ttttcagaac tttcctgtag actctgattg ggacgaagac 2220

cctgacttat tcttggaacg gttagctttc cctgaccttt ggcttcaccc tctgatacca 2280

actgattata ttaaaaactt accaatgtgg cgatttaaat gtcttggggt tcagtctgaa 2340

gaagaaatgt cagagagttc tcaagacact gaaaatgact cggatagtga caacatggac 2400

acttttagta gtagtaagga tatatttgaa gataaacaaa gcgttgttca cagttctcca 2460

ggaagaacaa gtcactgtga tactgaggct tgttcatgtg ccaataaatg tgagagcagc 2520

ccatgtgaaa ggaagaggag gtcgtatgga tcacataata ctgatgaaga tatggaaccg 2580

gactggttaa cttggcctgc gggtacgctg cactgtactg aatgtgttgt ttgccttgag 2640

aattttgaaa atggatgttt gctaatgggg ttgccttgtg gtcacgtgtt tcaccagaat 2700

tgcattgtta tgtggttggc tgggggccga cactgttgcc ctgtttgtcg ttggccttca 2760

tataagaaaa agcagccata tgcacaacaa cagccgttgt caaatgatgt tccatcttaa 2820

ccatgtgcaa tttgtcctta ataagccttg agtatcttac agcttgcctt tttaatgtta 2880

gtcacaatgt ttttgtggtt tgaagtttag tttaatgtta gtgcagtgac aggaaataca 2940

cattatgctg atgttgatga cagaatttat ttggttgcct tgtgtgtcaa ttgaatgcat 3000

actaaactgt aaaaaaaatt tatttacagc attgaaaatt cagaagttaa tggttttttg 3060

taagcacaaa agaagtatgg tagaaattaa tcttaacaag actttatgag gcaggatcaa 3120

atcctagtgg gcctgagcag gtttcttacc ctaagtgttt tctccctttt tacaatctct 3180

gtccagcacc tcttggttaa ataatgtatg ctctgagaca tgaaattaaa acagatctat 3240

aaaataaatt attttaaaag c 3261

<210> 8
<211> 23
<212> DNA
<213> Artificial

<220>
<223> primer

<400> 8
aacttgaagt cgctgtcttt tgg 23

<210> 9
<211> 24
<212> DNA
<213> Artificial

<220>
<223> primer

<400> 9
gaatgggctg accgcttcct cgtg 24

<210> 10
<211> 24
<212> DNA
<213> Artificial

<220>
<223> primer

<400> 10
tcttggttaa ataatgtatg ctct 24

<210> 11
<211> 23
<212> DNA
<213> Artificial

<220>
<223> primer

<400> 11
aacttgaagt cgctgtcttt tgg 23

<210> 12
<211> 24
<212> DNA
<213> Artificial

<220>
<223>  primer

<400>  12
cccctataaa attctttcct atcc                                              24

## Description

### TECHNICAL FIELD

The present invention relates mainly to a knockout nonhuman animal exhibiting anxiety-like behaviors and the usage thereof.

### BACKGROUND ART

The *kf-1* gene is highly expressed in the hippocampus and the cerebellum of a healthy human brain but barely expressed in the cerebral cortex. The present inventors identified two genes, which are expressed more frequently in the cerebral cortex of an Alzheimer's disease (AD) patient. One of the two genes was novel at that time. The novel gene was named *kf-1* (Non-Patent Document 1, Patent Document 1, etc.).

The other gene was the gene for glial fibrillary acidic protein (GFAP), which was conventionally known being expressed more in the cerebral cortex of Alzheimer's disease patients.

In order to clarify the function of *kf-1* gene, the present inventors attempted to create a knockout mouse using the Cre-lox conditional expression system.

Thereafter, another group (Department of Psychiatry, School of Medicine, Showa University) identified a gene having an enhanced expression in the cerebral cortex after chronic administrations of selective-serotonin-reuptake-inhibitor (SSRI), which is an antidepressant drug, and reported that the identified gene was an orthologue of the human *kf-1* gene (Non-Patent Document 2). The group also reported that antidepressive repeated electroconvulsive treatment showed similar augmentation (Non-Patent Document 3).
Patent Document 1: Japanese Unexamined Patent Publication No.9-215495
Non-Patent Document 1: Yasojima et al., 1997, BBRC, 231(2):481-487
Non-Patent Document 2: Yamada, M., et al., 2002, BBRC, 78(1):150-157
Non-Patent Document 3: Nishioka et al., 2003, J. Neural. Transm., 110(3):277-285

### DISCLOSURE OF THE INVENTION

### Problem to be solved by the Invention

An object of the present invention is to provide a nonhuman knockout animal that is useful for the elucidation of anxiety-like behavior, or the development of medicine for preventing, treating or alleviating anxiety-like behavior, and the method for using the animal.

### Means for Solving the Problem

The present inventors conducted extensive research on the behavioral aspects of *kf-1* knockout mice in order to elucidate the possible association of *kf-1* gene to neurological diseases.

Specifically, the present inventors constructed a vector having loxP sites in the *kf-1* gene, and subjected 432 clones of resulting G418-resistant ES cells to PCR-based screening analysis. Consequently, five homologous recombinant clones were obtained and chimeric mice were generated by using these ES cells. Thereafter, they were mated with Cre-expressing mice to produce *kf-1* null mice, and an analysis mainly of the effect on the nervous system was conducted.

As a result, increased anxiety-like behaviors were observed in anxiety-like behavior tests. The present invention has been accomplished by conducting extensive research based on this finding.

Specifically, the present invention provides the followings:

Item 1: A vector for creating a *kf-1* gene knockout nonhuman animal exhibiting anxiety-like behaviors comprising:

LoxP-pM-M-*kf-1*[in3b]-*kf-1*[ex4a]-LoxP

wherein M is a selection marker gene,
pM is a promoter for expression of the selection marker gene,
*kf-1*[in3b] is a base sequence represented by SEQ ID NO: 2, and
*kf-1*[ex4a] is a base sequence represented by SEQ ID NO: 3.

It is preferable that the pM-M in the vector of Item 1 be pgk-Neo, which is a pKJ2-derived neomycin resistance gene.

Preferably, the vector according to Item 1 has a base sequence represented by SEQ ID NO: 1 and/or SEQ ID NO: 4 located outside the regions flanked by a set of LoxP cassette.

Preferably, the vector according to Item 1 consists of a base sequence represented by SEQ ID NO: 6.

Preferably, a use of a vector comprising:

LoxP-pM-M-*kf-1*[in3b]-*kf-1*[ex4a]-LoxP

wherein M is a selection marker gene,
pM is a promoter for expression of the selection marker gene,
*kf-1*[in3b] is a base sequence represented by SEQ ID NO: 2, and
*kf-1*[ex4a] is a base sequence represented by SEQ ID NO: 3;
for creation or generation of a *kf-1* gene knockout nonhuman animals exhibiting anxiety-like behaviors.

Item 2: A *kf-1* gene knockout nonhuman animal (may be male or female) created by using the vector of Item 1 or a descendant of the animal.
Preferably, a *kf-1* gene knockout nonhuman animal (may be male or female) or a descendant thereof for use in screening of compounds for their potential to prevent, treat or alleviate an anxiety disorder, or anxiety-like behaviors as a part of depression or other psychiatric diseases.

Item 3: A modified animal derived from the *kf-1* gene knockout nonhuman animal or a descendant thereof of item 2, or a descendant of the modified nonhuman animal.
Preferably, a modified nonhuman animal lacking *kf-1* gene or a descendant thereof or a descendant of the modified nonhuman animal, wherein the *kf-1* gene knockout nonhuman animal(may be male or female) is usable for screening compounds or their potential to prevent, treat or alleviate an anxiety disorder, or anxiety-like behaviors as a part of depression or other psychiatric diseases, particularly, the genuine anxiety disorder.

Item 4: A method for screening compounds for their potential to prevent, treat or alleviate an anxiety disorder or anxiety-like behaviors as a part of depression or other psychiatric diseases,
the method comprising Steps (1) to (3) below:
(1) administering a test compound to the nonhuman animals or descendants thereof of Item 2 or 3;
(2) observing or measuring the anxiety-like behaviors of the nonhuman animal group before and after the administration of the test compound or observing or measuring anxiety-like behaviors of the nonhuman animal group to which the test compound is administered and that of a placebo administration group; and
(3) comparing the results in Step (2) to select a test compound that can decrease anxiety-like behaviors.

The method of Item 4 for screening compounds for their potential to prevent, treat or alleviate anxiety-like behaviors,
preferably having Steps (1) to (3) below:
(1) administering a test compound or placebo to the nonhuman animals or descendants thereof of Item 2 or 3;
(2) observing anxiety-like behaviors of the nonhuman animal group to which the test compound is administered and that of the group to which the placebo is administered; and
(3) comparing the results in Step (2) to select a test compound that can suppress anxiety-like behaviors.

Two similar groups using wild-type mice may also be added to the above steps.

Item 5: A medical composition for preventing, treating or alleviating an anxiety disorder or anxiety-like behaviors that is a part of depression or other psychiatric diseases,
the medical composition comprising the compound selected for the first time by the screening method of Item 4 as an active ingredient.

Among the medical composition of Item 5, a composition for preventing, treating or alleviating, in particular, an anxiety-like behaviors, which contains the compound selected by the screening method of Item 4 as an active ingredient.

The present invention is explained in detail below.

### 1. Vector

The vector of the present invention is desirably usable for creating a knockout nonhuman animal exhibiting anxiety-like behaviors.
The structure of the vector is LoxP-pM-M-*kf-1*[in3b]-*kf-1*[ex4a]-LoxP,
wherein M is a selection marker gene,
pM is a promoter for the selection marker gene expression,
*kf-1*[in3b] is a base sequence represented by SEQ ID NO: 2, and
*kf-1*(ex4a) is a base sequence represented by SEQ ID NO: 3.
Here, the *kf-1*[in3b] is a part of the intron 3 of a mouse *kf-1* gene. The *kf-1*[ex4a] is a large part of the exon 4 of the mouse *kf-1* gene. The LoxP denotes the LoxP sequences used for a Cre-LoxP system.

The selection marker gene and the promoter for its expression maybe suitably chosen from known ones. A preferable example thereof is the pgk-driven neomycin (G418) resistance gene, which is derived from pKJ2, resulting in pgk-Neo.

The vector in the present invention may have a *kf-1* intron or exon, or a part thereof, outside of the region flanked by the LoxP cassettes that are inserted at the both ends. For example, the vector may have base sequences represented by SEQ ID NO: 1 and/or SEQ ID NO: 4.

One example of the preferable vectors of the present invention includes a vector consisting of the base sequences represented by SEQ ID NO: 6.

As long as the effect of the present invention can be maintained, the gene can be modified appropriately or a suitable linker may be added to the vector of the present invention.

The vector of the present invention may be suitably constructed by a known method, for example, the method described in Example 1.

By using the vector of the present invention with the Cre-LoxP system, a *kf-1* gene knockout animal exhibiting anxiety-like behaviors can be reliably produced.

### 2. Knockout Nonhuman Animals or Its Descendants

In the present invention, the term of "knockout nonhuman animals" includes any animals other than humans. A usable animal is not limited to the vertebrates. Examples of the animals include mouse, rat, rabbit, guinea pig, swine, sheep, goat, etc. Among these, a mouse is preferably used in the present invention. A "descendant" animal can be generated by the standard method such as mating using the knockout nonhuman animals as parents or ancestors.

The present invention is explained in detail below using a mouse as a sample animal.

### 2-1. Creation of Knockout Nonhuman Animals

The knockout nonhuman animals of the present invention can be created using the above-mentioned target vector with the Cre-LoxP system. Specifically, the vector is produced by inserting a *kf-1* gene or a part thereof between two LoxP sequences. The thus-obtained target vector is introduced into ES cells. Resistant clones can be obtained by culturing cells introduced with the target vector in the presence of an appropriate drug, for example, G418. Thereafter, desired clones having homologous recombination are selected by, for example, southern blot analysis, and microinjected into pregnant mice with early embryos to obtain a chimeric mouse. The thus-obtained chimeric mouse is crossed with the wild-type mouse to obtain a heterozygous mouse. The thus-obtained heterozygous mouse is mated with another heterozygous mouse similarly obtained to obtain a homozygous mouse.

Specifically, the knockout nonhuman animal of the present invention can be produced by, for example, the method disclosed in Example 1.

### 2-2. Characteristics

The knockout nonhuman animal of the present invention or a descendant thereof exhibits anxiety-like behaviors.

In the present specification, the term "anxiety-like behaviors" means behaviors associated with anxiety.

In the present invention, the term "exhibiting anxiety-like behaviors" indicates that, in behavioral anxiety tests, mice exhibit remarkably increased anxiety-like behaviors than the wild-type or normal mice (hereunder, they are referred to as the control group or control mice). In particular, it means that the mice display significantly increased anxiety-like behaviors but do not exhibit significant differences from the control group in general locomotor abilities or other behavioral activities, learning ability, depression-like behaviors defined by depression model experiments such as the forced swim test, and social activities defined by the social behavior test.

"Depression" model experiments mentioned above include, for example, the forced swim test and/or tail suspension test. Examples of anxiety-like behavior tests include the light/dark transition test, and the elevated plus-maze test and/or the startle-response test (prepulse inhibition test).

The experimental results of the knockout nonhuman animals of the present invention in the forced swim test, which is one of the "depression" model tests, were negative, i.e., there is no significant difference from the results of the control group. However, in the light/dark test and/or elevated plus-maze test, which are anxiety-like behavior tests, remarkably enhanced anxiety-like behaviors are observed, i.e., the knockout nonhuman animal of the present invention exhibits significantly increased anxiety- but not depression-like behaviors than the control group. The knockout nonhuman animals of the present invention exhibit an elevated sensorimotor gating ability in startle response test (prepulse inhibition test), which examines the control of sensory-information filtering. An animal or human suffering from schizophrenia is defective in sensorimotor gating ability to filter out unnecessary sensory-information.
However, the knockout mice do not exhibit such a symptom as observed in schizophrenic animals and humans, but exhibit the increased ability for suppressing the startle response in the prepulse inhibition test than the wild-type mice do.
This seems to be probably because suppression of the startle response may be relevant to anxiety-like behaviors triggered by an increase in sensitivity to sensory information.

Such characteristics of the knockout mice of the present invention indicate that they can be used as an animal model for some human mental disorders, for example, social withdrawal disorder, which is pointed out to be one of the depressive symptoms and a heralding symptom or complication of Alzheimer-type dementia, although the detailed entity of these symptoms are not yet clarified.

Specifically, the knockout nonhuman animal of the present invention is usable as a model animal for research to elucidate the control mechanisms of anxiety-like behaviors including behavioral disorders that are considered to consist of multiple symptoms of depression, for example, anxiety, decreased libido, social withdrawal, social isolation, suicidal ideation, etc. The knockout nonhuman animal of the present invention is also usable as an experimental animal for screening compounds for potential to prevent, treat or alleviate anxiety-like behaviors.

The knockout nonhuman animal of the present invention or a descendant thereof has, for example, the following characteristics:
Genetic Classification: Targeted Mutation Congenic Origin of the strain: The mouse was created by the introduction of DNA having LoxP both before and after the exon 4 of the mouse *kf-1* gene to mouse embryonic stem cells. The resulting mouse was mated with a Cre Tg mouse, resulting in a mouse and descendants thereof in which the *kf-1* exon 4 was deleted.
Microbiological Breeding Environment: conventional
Microbiological Characteristics: ICLAS (microscopic examination I, culture I, serum I) negative, S. aureus positive
Details of the strain (characteristics and use): Exhibiting anxiety-like behaviors similar to symptoms observed in "social withdrawal"
Breeding and Mating: Reproductive efficiency A
Remarks: Abnormal behaviors were observed. Anxiety-like behaviors or behaviors similar to that observed in symptoms of "social withdrawal disorder" was exhibited in the light/dark test and elevated plus-maze test. However, "depression-like" symptoms defined by the forced swim test and/or tail suspension test were not observed.
In contrast with schizophrenia, the *kf-1* knockout mice exhibit significantly increased ability of sensorimotor gating and increased inhibition of the startle response compared to the wild-type mice.

The present invention includes the knockout nonhuman animal produced in the manner described above or its modified descendant. The modified animals include any one that is modified by genetic manipulation, mating, etc., or a descendant thereof.

### 3. Screening Method

The present invention provides a method for screening compounds, by using the knockout nonhuman animal, which are useful for prevention, treatment or alleviation of anxiety disorders or anxiety-like behaviors that constitute a basic disorder of depression or other psychiatric diseases.

Specifically, the present invention provides a method for screening compounds, using the knockout nonhuman animal, which are useful for the prevention, treatment or alleviation of an anxiety disorder or anxiety-like behaviors that constitute a basic disorder of depression or other psychiatric diseases. The method includes the following Steps (1) to (3).
Step (1): Administering the test compound to the nonhuman animals or descendants thereof;
Step (2): Observing and measuring the anxiety-like behaviors of the nonhuman animals in the group to which the test compound was administered and the group to which a placebo was administered; and
Step (3): Selecting a test compound that can alleviate anxiety-like behaviors by comparing the results of the observation and measurement in Step (2).

There is no limitation to the administration method of the test compound and a standard method can be employed.

There is no limitation to the means for measuring anxiety-like behaviors, and the measurement can be conducted by an anxiety-like behavior test using a known method.

Examples of anxiety-like behavior tests include the light/dark test, elevated plus-maze test, prepulse inhibition test, etc.

The light/dark test is conducted according to the following procedure. A mouse is first placed in a dark compartment of the light-dark boxes having a dark compartment and light compartment communicably adjacent to each other, followed by measurements, within a predetermined time, of (1) the duration of time that the mouse stays in the light compartment and the duration of time that the mouse stays in the dark compartment; (2) the number of transition times the mouse traveled between the two compartments; (3) the latency time until the mouse enter the light compartment for the first time; (4) the total distance traveled in respective compartments; etc.

It is determined that anxiety-like behaviors increase if the time spent in the light compartment is shortened, the number of times traveled between the compartments decreases, or the latency time before going into the light compartment for the first time is prolonged.

The elevated plus-maze test is conducted in the following procedure. A cross-shaped elevated maze (having two open arms without walls and two closed arms with walls) is prepared. A mouse is placed on the platform in the crossing center of the maze. Measurements are conducted within a predetermined time regarding (1) the duration time that the mouse stays in the open arms and the duration time that the mouse stays in the closed arms; (2) the respective number of times that the mouse enters the open and closed arms; (3) the total traveling distance on the respective arms; etc.

If the time spent in the open arms is prolonged, it is determined that the anxiety-like behaviors are decreased. In contrast, if the stay time in the open arms is shortened, it is determined that the anxiety-like behaviors are increased.

The prepulse inhibition test is conducted in the following procedure. Mice are placed in a test box equipped with a device for measuring a change in load for detecting a startle reaction. When the mice become acclimated to the surroundings of the device, the duration of white noise is used in the box for adaptation of mice to the new atmosphere. The magnitude of the startle responses are expressed as an increase in load when a strong acoustic stimulus is given suddenly, or when strong acoustic stimulus is given after the prepulse stimulus, i.e., A and B successively. The reduction ratio of the startle response (prepulse inhibition) C (%) can be obtained by C = 100 (1-B/A).

When the load resulting from a response to strong acoustic stimulus decreases compared to that resulting from a preceding acoustic stimulus, i.e., when the value of C increases, it is assumed that the startle response is reduced. It is known that the reduction in the startle response is significantly lower in a schizophrenic group compared to that of the control group, probably because the schizophrenic group has disorders in sensorimotor gating. If the reduction of the startle response is significantly higher than that of the control group, they have reinforced sensorimotor gating, i.e., it reflects the condition where the anxiety-like behaviors are increased.

Regarding this behavioral test, if the knockout nonhuman animals being administered the test compound exhibit alleviation or prevention of anxiety-like behaviors compared to the group to which a placebo was administered, the test compound can be identified as a compound or a candidate compound that is useful for prevention, treatment or alleviation of an anxiety disorder or anxiety-like behaviors that are a part of depression or other psychiatric diseases.

Examples of anxiety disorders associated with psychiatric diseases other than anxiety disorder associated with depression, include anxiety disorders attributable to schizophrenia, dementia, social withdrawal disorder, etc., but not limited to these.

### 4. Medical Composition

The medical composition of the present invention may contain a compound selected by the above-explained screening method as an active ingredient.

In particular, the present invention provides a medical composition that contains, as an active ingredient, the compound that was selected according to the above-described screening method. The medical composition of the present invention can prevent, treat or alleviate an anxiety disorder or anxiety-like behaviors that are a part of depression or other psychiatric diseases.

The medical composition obtained by the present invention may contain appropriate pharmacological carriers, various excipients generally blended with a medicinal composition, and other medicinal properties as long as they do not adversely affect the effects of the present invention.

There is no limitation to the production method of the medical composition of the present invention; it can be appropriately produced in accordance with a publicly known procedure.

The medical composition obtained by the present invention contains, as an active ingredient, the compound that was selected for the first time by the above-described screening method. The medical composition can prevent, treat or alleviate an anxiety disorder or anxiety-like behaviors that are a part of "depression" or other psychiatric diseases. Hereunder, the mouse "depression" symptom that is determined by the forced swim test and/or tail suspension test is expressed using quotation marks, i.e., "depression", so as to distinguish it from human depression that is clinically diagnosed.

Specifically, the pharmacological compounds obtained by the present invention can be used for preventing, treating or alleviating an anxiety disorder or anxiety-like behaviors as one of the symptoms of depression such as fear, decreased libido, social withdrawal, social isolation, suicidal ideation and behavioral disorders resulting thereof, which are caused by emotional drive seen generally in depression.

### EFFECT OF THE INVENTION

Currently, it is becoming clear that there is a large difference based on gender in humans at the onset of the symptoms of "social withdrawal disorder" (about 80% withdrawers are male), and many suffering from symptoms of "social withdrawal disorder" have relatives with a similar medical history, etc. Therefore, it is believed that a biological factor may be involved in the onset of the symptoms of "social withdrawal disorder".

In order to develop a medicine that is effective for alleviating the symptoms of "social withdrawal disorder", it is necessary to use a simple animal (e.g., mouse) that exhibits the symptoms of "social withdrawal disorder" and like anxiety disorders, but the presence of such an animal has not been reported yet.

The *kf-1* null mouse developed in the present invention does not exhibit the symptoms of "depression" that are defined by the forced swim test and/or the tail suspension test. However, the *kf-1* null mice of the present invention specifically exhibit anxiety-like behaviors defined by the elevated plus-maze test and the light/dark test. In other words, the *kf-1* null mouse of the present invention exhibits behaviors similar to that observed in "social withdrawal" symptoms.

These characteristics of social withdrawal-like behaviors are believed to be some of the symptoms of depression, a prodrome of Alzheimer's disease, etc. However, they may be associated with complications of various psychoneuroses of which details have not been substantively elucidated, for example, anxiety disorder, decreased libido, social isolation, suicidal ideation and like behavioral abnormalities.

Therefore, the *kf-1* null mouse of the present invention may be usable as an effective means for elucidating various psychoneuroses, for example, anxiety disorder, decreased libido, social isolation, suicidal ideation and like depression-like behavioral abnormalities and symptoms of "social withdrawal disorder". The *kf-1* null mouse of the present invention may also be usable as an effective means for developing or screening a medical composition that can substantively in the prevention, treatment or alleviation of such symptoms.

### BRIEF DESCRIPTION OF THE DRAWINGS

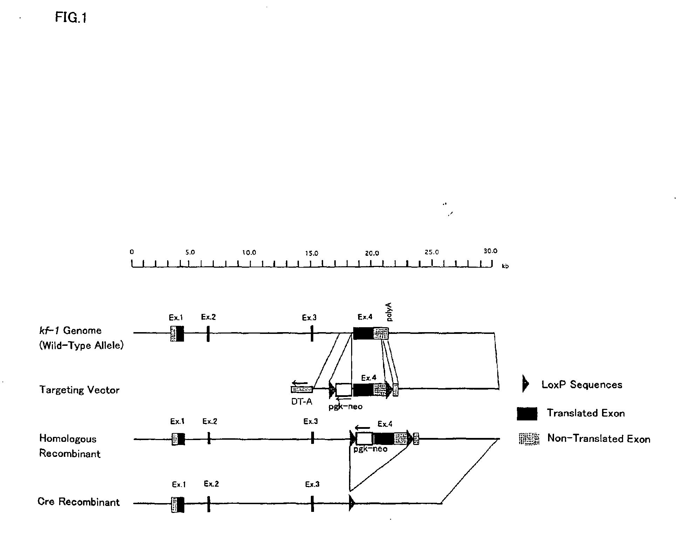

FIG. 1 explains the gene targeting method used in the production of a *kf-1* knockout mouse in the Example. The figure schematically shows the structures of, in order from the top, a *kf-1* gene (wild-type allele) region in a normal chromosome, a targeting vector for the production of a *kf-1* knockout mouse, a *kf-1* gene that has undergone homologous recombination and into which a loxP site and a neomycin (G418) resistance gene have been inserted (homologous recombinant), and a mutant allele (Cre recombinant) from which a large portion of an *kf-1* gene-translation region was deleted a result of a cross with a transgenic mouse expressing Cre.

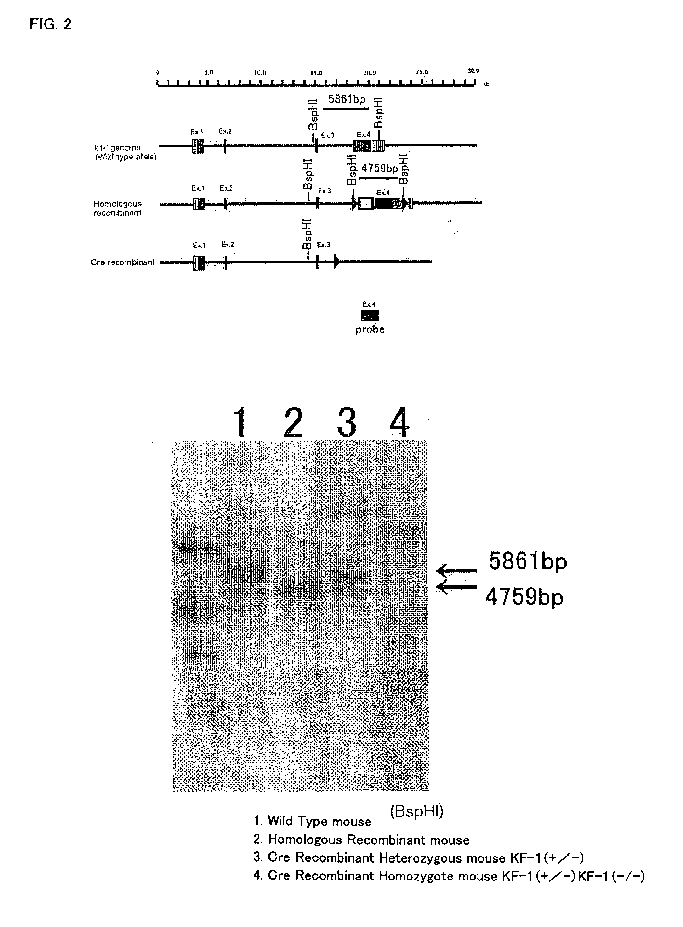

FIG. 2 shows the results of the Southern blotting analysis of the genomic DNA isolated from a wild-type mouse, a homologous recombinant mouse, and a homozygous Cre recombinant mouse (*kf-1*(-/-)). The upper panel shows the structure of, from the top, the wild type *kf-1* gene, recombinant *kf-1* gene with a targeting vector and *kf-1* mutant allele lacking exon 4 as a result of crossing with a Cre expressing transgenic mouse with the BspHI restriction sites, and the probe. The lower panel shows the results of Southern hybridization experiment conducted using the exon 4 of mouse *kf-1* as a probe for BspHI-digested genomic DNAs derived from the wild-type mouse, a homozygote mouse with homologous recombination with the targeting vector, a heterozygote mouse having a *kf-1* gene in which exon 4 is deleted in one of the alleles as a result of crossing with a Cre-expressing transgenic mouse, and a *kf-1*(-/-) homozygote mouse having no exon 4 in either allele of *kf-1* gene. The lane 4 indicates that the exon 4 is completely lost in the *kf-1* (-/-) mouse.

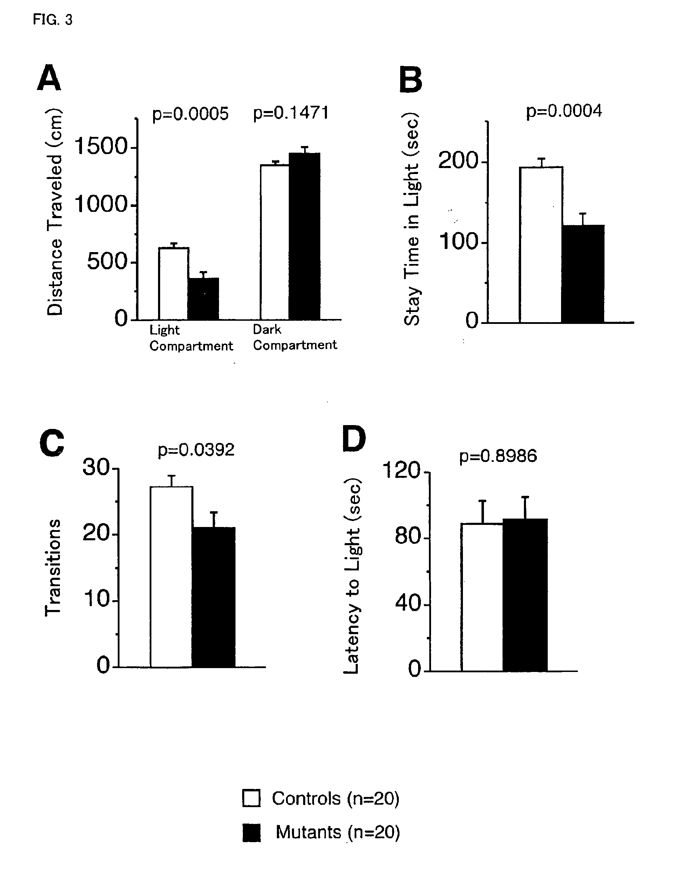

FIG. 3 shows the results of light/dark transition test in *kf-1* (-/-) mice produced in Example 1. The wild-type mice (Controls) and *kf-1* null mice (Mutants) were placed in a dark compartment at the beginning, and A: the distance traveled within the light compartment and the dark compartment, B: the stay time in the light compartment, C: the number of transitions between the light compartment and the dark compartment, and D: the latency time before the first entering into the light compartment were measured. The results of the significance test using a variance analysis indicated that mutant mice exhibited a significantly lower level of locomoter activity merely in the light compartment compared to that of the wild type (A) and remarkably shorter stay time in the light compartment than the wild-type mice did (B); however, there were no significant differences in the distance traveled between the two groups in the dark compartment. There was also no significant difference between the *kf-1* null mice and the wild-type mice in the latency time spent until entering the light compartment for the first time.
   From these results, it can be concluded that there is no significant difference between the mutant mice and the wild-type mice in general and exploratory locomoter activities.; however, the mutant mice exhibited remarkably lowered locomoter activity in the light compartment. Because a significant difference was not observed in the time spent before entering the light compartment for the first time, it is clear that mutant mice are not suffering from remarked photophobia.

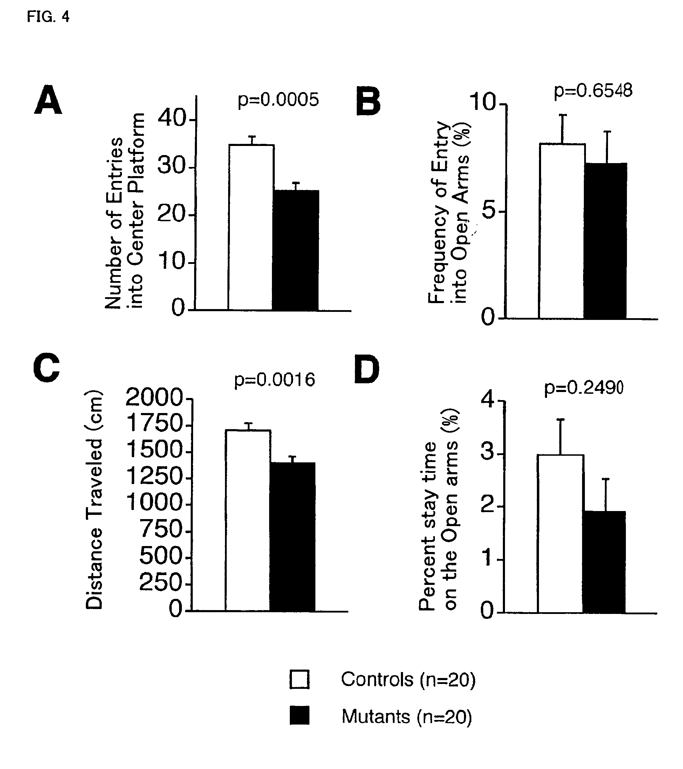

FIG. 4 shows the results of the elevated plus-maze test of *kf-1* (-/-) mice that were produced in Example 1. The wild-type mice (Controls) and *kf-1* null mice (Mutants) were placed individually at the center of the elevated plus-maze (center platform), and its activities were recorded for 10 minutes. This figure shows A: the number of entries into the center platform, B: the number of entries into the open arms, C: the total distance traveled, and D: percent of the stay time spent on the open arms. Mice inherently have acrophobia and tend to avoid the open arms. There was no significant difference between the wild-type mice and the mutant mice in this tendency (B, D). However, there were significant differences between the wild-type mice and the mutant mice in the frequency of entering the center of the plus-maze where the open arms cross with the closed arms (A), and the total distance traveled (C). It become clear that mutant mice tend to remain staying on either arm.

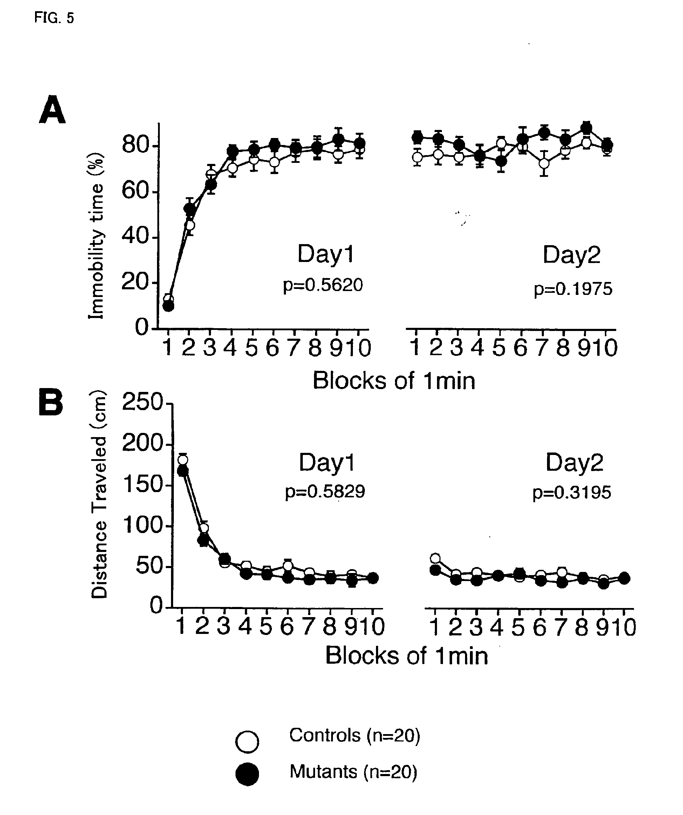

FIG. 5 shows the results of the forced swim test in *kf-1* (-/-) mice that were produced in Example 1. The wild-type mice (Controls) and *kf-1* null mice (Mutants) were placed individually in a water bath and their movements were recorded for 10 minutes. The graphs show the percent immobility time per minute (A), and the distance traveled every minute (B) (Day 1). The same test was repeated after 24 hours, and the results are shown as Day 2. In the forced swim test, there were no significant differences between the wild-type mice and the *kf-1* (-/-) mice, and a lack of eagerness to live is not observed, which is an indication of the onset of "depression" symptoms in mice.

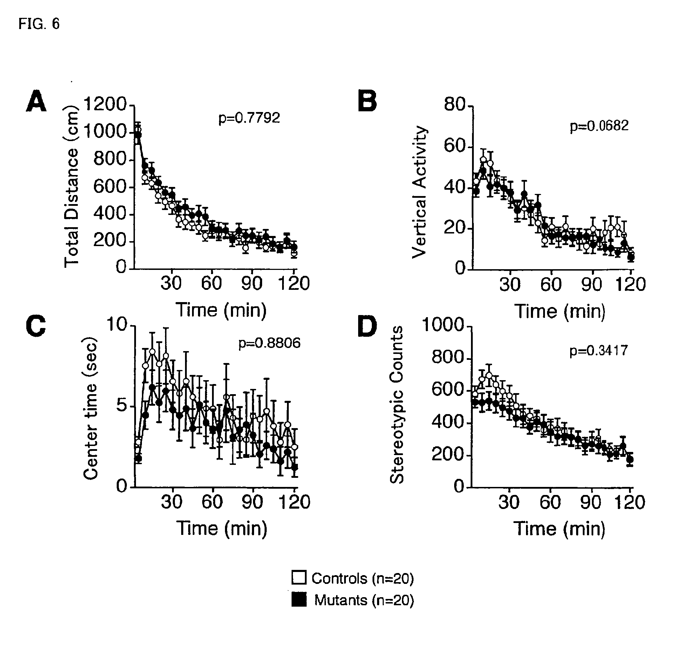

FIG. 6 shows the open-field-test results of the *kf-1* (-/-) mouse produced in Example 1. The actions of wild-type mice (Controls) and *kf-1* null mice (Mutants) on the open field were observed for 120 minutes, and total locomotion distance per 5 minute interval (A), count of vertical activity per 5 minute interval (B), the time spent in the center of the compartment per 5 minute interval (C), and count of stereotypic behaviors per 5 minute interval (D) was scored. There were no significant differences between the wild-type mice and the *kf-1* (-/-) mice in their behavioral pattern. It is clear that a significant reduction of activity was not observed in the *kf-1* null mice.

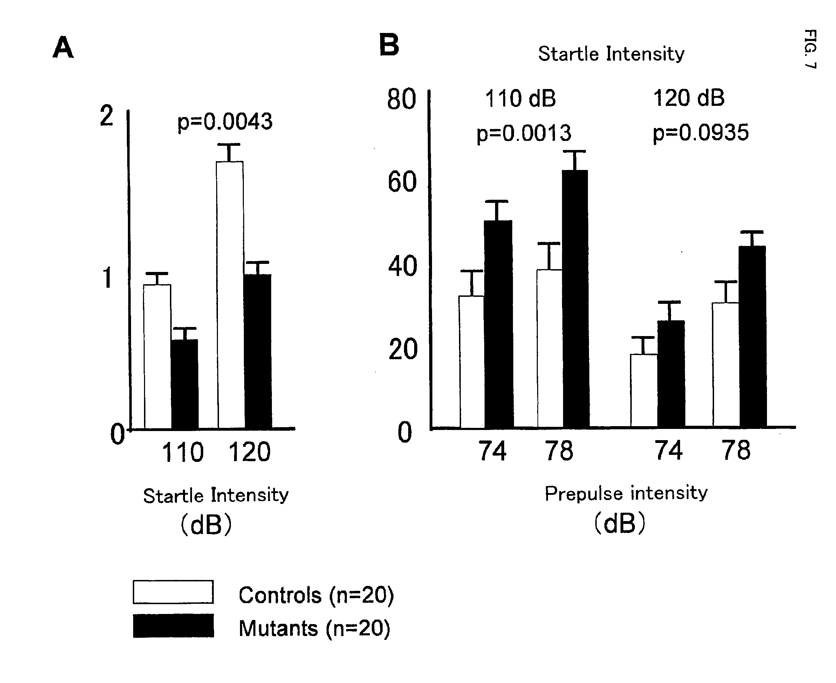

FIG. 7 shows the results of prepulse inhibition test in the kf-1 (-/-) mouse produced in Example 1. FIG.7(A) shows that startle response amplitude of mutant mice is significantly reduced compared to that of the wild-type mice (Controls), which is shown as a ratio of the weight loaded upon startle response to the weight of a resting mouse at the time of acoustic stimulus without preceding stimulus (110 dB or 120 dB). This corresponds to an increase in their anxiety-like behaviors. FIG. 7(B) shows that percent prepulse inhibition was significantly increased in kf-1(-/-) compared to that in the wild-type mice upon strong acoustic stimulus following the weak acoustic stimulus in comparison with that upon strong acoustic stimulus alone. Compared to the wild-type mice, the *kf-1* (-/-) mice exhibited better learning effects after receiving the preceding stimulus. This indicates that the increased prepulse inhibition of the startle response corresponds to increased anxiety-like behaviors.

### BEST MODE FOR CARRYING OUT THE INVENTION

The Examples of the present invention are explained in detail with reference to the attached drawings. However, the scope of the present invention is not limited to these Examples.

### EXAMPLES

### 1. Creation of the kf-1 Knockout Mouse

### [1.1]

In order to construct the targeting vector, using mouse *kf-1* cDNA shown by SEQ ID NO: 7 in the Sequence List as a probe, a mouse genomic DNA library was screened by plaque hybridization, and several genomic DNAs containing the mouse *kf-1* gene were isolated. The mouse *kf-1* gene consist of four exons ranging over approximately 20 Kb. By using the obtained mouse *kf-1* gene DNA, a targeting vector was constructed as to line up from left to right, 1.9 Kb long AseI-StuI fragment located in the Intron 3 (Intron 3a) as the upstream arm, LoxP sequence, Neomycin resistant gene derived from pKJ2, 2.6 Kb long StuI-BglII fragment including Intron 3 (intron 3b) from the StuI site to exon 4 and the exon 4 from the beginning of it to the BglII site(exon 4a), LoxP sequence, and exon 4 from the BglII site to the polyA additional site (exon 4b) followed by the genomic downstream region as the downstream arm. A diphtheria toxin gene (DT-A) was placed in front of the upstream arm segment as a genetic marker for negative selection against clones without homologous recombination, and the resulting construct was used as the targeting vector (FIG. 1).

Note that pHSG396 (disclosed in Gene, 1987; 61:63-74.) was used for the backbone of this targeting vector.

Complete sequences of the thus-constructed targeting vector are shown by SEQ ID NO: 6 in the Sequence List.
Each region of the transgenic vector is explained below.
Region Source
1-1206 pHSG396
1218-2772 pMC1_DTpA(diphtheria toxin gene)
2780-4695 mouse *kf-1* gene intron 3a
4759-4942 pBS246 (LoxP cassette)
4997-6329 pKJ2 (pgk-Neo)
6376-6956 mouse *kf-1* gene intron 3b
6957-8943 mouse *kf-1* gene exon 4a
8959-9037 pBS246 (LoxP cassette)
9044-9074 mouse *kf-1* gene exon 4b
9075-19222 mouse genome downstream
19223-19252 Linker
19562-20248 pHSG396.

The mouse *kf-1* gene intron 3a had the sequence represented by SEQ ID NO: 1 in the Sequence Listing.
The mouse *kf-1* gene intron 3b had the sequence represented by SEQ ID NO: 2 in the Sequence Listing.
The mouse *kf-1* gene exon 4a had the sequence represented by SEQ ID NO: 3 in the Sequence Listing.
The mouse *kf-1* gene exon 4b had the sequence represented by SEQ ID NO: 4 in the Sequence Listing.
The downstream region after mouse kf-1 gene has the sequence represented by SEQ ID NO: 5 in the Sequence Listing.

### [1.2]

After linearization, the above-mentioned targeting vector was introduced into 129SVJ embryonic stem cells by electroporation. Clones resistant to antibiotic G418 were selected. Resulting 432 clones were subjected to PCR analysis using the two types of primers described below, and five homologous recombinant clones were obtained producing 483 bp long amplified fragment.
mgKF_U17436 :5'-AACTTGAAGTCGCTGTCTTTTGG (the sequence represented by SEQ ID NO: 8),
Neo-F712 :5'- GAATGGGCTGACCGCTTCCTCGTG (the sequence represented by SEQ ID NO: 9).

### [1.3]

Chimeric mice were obtained by microinjecting the homologous recombinant ES clones into mouse early embryos by the known method (Genes Dev. (1994) 8, 707-719). The thus-obtained chimeric mice were crossed with wild-type C57BL/6 mice, and the resulting heterozygous male mice were crossed with Cre-expressing female mice, and then heterozygous mice with deletion of *kf-1* Exon 4 were obtained. By crossing between male and female heterozygous mice, *kf-1* knockout mice having homozygous deletion alleles (hereunder the mouse is referred to as a *"kf-1*(-/-) mouse") were produced.

After having isolated genomic DNA from each mouse and having digested it with BspHI restriction enzyme, Southern hybridization was conducted using exon 4 of *kf-1* gene as a probe. Consequently, a band of about 5.86 kb was observed in the wild type C57BL/6, and a band of about 4.76 kb was observed in the homologous recombinant mouse. In the Cre recombinant heterozygous mouse, the intensity of 5.86 Kb long DNA fragment decreased to a half of that obtained in the wild type, and no *kf-1* Exon 4 bands were detected at all in the Cre recombinant homozygous mouse (*kf-1* (-/-)) (FIG 2).

Furthermore, using the primers shown below, PCR was conducted.
20039FW: TCTTGGTTAAATAATGTATGCTCT (the sequence represented by SEQ ID NO: 10)
17436FW: AACTTGAAGTCGCTGTCTTTTGG (the sequence represented by SEQ ID NO: 11)
20292RV: CCCCTATAAAATTCTTTCCTATCC (the sequence represented by SEQ ID NO: 12)
The results show that, the amplified product in the wild-type (WT) mouse was a 277 bp long fragment and the amplified product in the knockout mouse (Hicky) was a 560 bp long fragment.

There were no significant differences between the *kf-1*(-/-) mouse and the wild-type mouse in appearance and reproductivity.

### [1.4]

The obtained F1 *kf-1*(+/-) mice were repeatedly backcrossed in total 8 times to C57BL/6N. The resulting male and female *kf-1*(+/-) F9 mice were intercrossed with each other, and 20 male littermates of *kf-1*(+/+) and *kf-1*(-/-) (40 mice in total) were obtained. When the mice became four-weeks old, mice were group-housed with two *kf-1*(+/+) and two *kf-1*(-/-) littermates in one cage up to ten weeks old, and then used in the following behavioral experiments.

### 2. Behavioral Experiments of kf-1(-/-) Mice

The significant difference test was conducted using the variance analysis method. When p<0.05, it is judged that there is a significant difference.

### [2.1] Light/Dark transition Test

Whether or not mice display an increased anxiety was determined according to the anxiety disorder assessment method using light and dark boxes (Psychopharmacology, 94, 392-396, 1988). The experimental apparatus consists of two compartments, one is a dark compartment and the other is a light compartment. The light compartment was illuminated brightly with a lamp, and the dark compartment was connected next to the light compartment by tunnel. The test animal was placed first in the dark compartment and its behaviors were recorded for 10 minutes using a video camera installed in the lid of a box. The distance traveled within each box (Distance Traveled), the time spent in the light compartment (Stay Time in Light), the number of times traveled between the dark compartment and the light compartment (Transitions), and the time elapsed until the mouse enters the light compartment for the first time (Latency to Light) from the dark compartment were analyzed.

FIG.3 shows the results.

The results show that there were no significant differences between the two groups in the total distance traveled within the dark compartment and the time elapsed before entering the light compartment from the dark compartment (Latency to Light).
However, in distance traveled in the light compartment, the time spent in the light compartment, and the number of transitions between the dark compartment and the light compartment, *kf-1*(-/-) mice showed significantly reduced outcomes compared to the control mice. In other words, increased anxiety was aroused in *kf-1*(-/-) mice compared with the control mice.

### [2.2] Elevated Plus-Maze Test

Rising anxiety in the *kf-1*(-/-) mouse was determined according to the anxiety-like behavior assessment method using an elevated plus-maze (Miyakawa T, et al., Proc Natl Acad Sci USA. 2003; 100:8987-8992.).

The plus-maze equipment has two open arms without walls and two closed arms with walls of the same size; and the plus maze is elevated. The arms are connected to a central square so as to obtain a cross-shaped maze. Each mouse was placed at the center platform and the monitoring was commenced. The following actions were recorded for 10 minutes: the number of entries into the center platform (Number of Entries), the frequency of entering into the open arms (Entries into Open Arms %), the total distance traveled (Distance Traveled) and the percentage of time spent in the open arms (Times on Open Arms).

FIG. 4 shows the results.

The results showed that the number of entries into the center platform of the cross-shaped maze equipment and the total distance traveled were significantly reduced in *kf-1*(-/-) mice compared to the wild type mice. The number of entries onto open arms and stay time on the open arms were also reduced in mutant mice, but not significantly.

### [2.3] Forced Swim Test

Mouse "depression" symptoms were examined in terms of the lack of eagerness to live assessment, which is an indication of "depression" symptoms in mice by using a forced swim test (Arch. int. Pharmacodyn. 229, 327-336, 1977). The experimental equipment has four plastic cylindrical tanks with water therein. A mouse was placed in one of the tanks and its behaviors were recorded for 10 minutes. The analysis was conducted based on the result of measurements of immobility time per minute and the distance traveled per minute. The same experiment was repeated on the following day.

FIG. 5 shows the results.

The results show there was no significant difference between the *kf-1*(-/-) mice and the wild-type mice in terms of the immobility time and the distance traveled.

### [2.4] Open Field Test

Using the open field method, exploratory locomotion, general activity, and emotional behaviors of *kf-1*(-/-) mice were tested. The test animals were placed individually in a white acrylic cage, and the distance traveled (Total Distance), the number of times of rearing (Vertical Activity), the time spent in the central part (Center time), and the stereotypical behaviors (Stereotypic Counts) were measured, and changes in behaviors are shown at five-minute intervals on a graph.

FIG. 6 shows the results.

There were no significant differences between the *kf-1*(-/-) mice and the control group mice in all categories.

### [2.5] Startle Response/Prepulse Inhibition Test

The prepulse inhibition test was used to detect dysfunction, if any, of sensorimotor gating, as observed usually in schizophrenic patients, in the *kf-1*(-/-) mice. When only a strong acoustic stimulus is given, the normal sensory information process is conducted as to produce a startle reaction. However, when a preceding weak acoustic stimulus is given in advance, the startle reaction is weakened. When the *kf-1*(-/-) mice were given strong acoustic stimulus (110dB, 120dB), they exhibited a significantly weaker startle reaction compared to the control mice. However, when a preceding weak acoustic stimulus (74dB, 78dB) was given in advance, they exhibited a significantly increased inhibition of the startle response than the control mice.

The analysis was conducted as described below. A mouse was placed in a test box for detecting a startle reaction equipped with a change of load measuring device. The mouse was allowed to adapt to the surroundings of the device for 10 minutes, and white noise background was presented in the box for 5 minutes to make the mouse adapt to the new atmosphere. The magnitudes of the startle reactions, when only strong acoustic stimulus (110 dB, 120 dB) was given, or when strong acoustic stimulus was given after giving preceding soft acoustic stimulus (74 dB, 78 dB) were expressed as an increase in load, i.e., A and B respectively. The reduction rate of the startle reaction (prepulse inhibition) C% can be obtained by the equation of C = 100(1-B/A).

The results indicate that the *kf-1*(-/-) mice exhibited significantly lower startle reactions than the control mice when only strong acoustic stimulus was given. They also exhibited a significantly increased inhibition to startle reactions when strong acoustic stimulus was preceded by weak acoustic stimulus compared to the control mice. This may be interpreted that the *kf-1*(-/-) mice had a better learning ability, which is a reaction opposite to that observed in schizophrenia, and therefore it can be interpreted that the sensorimotor gating ability is augmented. The fact that the increased inhibition of startle response is associated with the enhancement of the anxiety-like behaviors in *kf-1* (-/-) mice as observed in the elevated plus-maze test or the light/dark test indicates that depression and schizophrenia are caused by abnormalities of the same function, and the abnormality may be expressed in opposite directions, i.e., dysfunction or enhancement of the functional ability. This implies that the increase in anxiety-like behaviors may be detected by not only observing anxiety-like behaviors itself using the elevated plus-maze test and the light/dark test but also by measuring the suppression of the startle response using the prepulse inhibition test. The prepulse inhibition test can provide a simpler screening procedure as to anxiety-like behaviors.

### [2.6] Results Analysis

There were no significant differences observed between *kf-1* null mice and the wild-type mice in body weight, the muscle strength test, etc. Furthermore, the results of the open-field test indicate that there was no significant difference in the general locomotor activity between the two groups. The results of the forced swim test show that so called mouse "depression" symptoms were not found in *kf-1* null mice.

However, in the light/dark transition test and the elevated plus-maze test, it was confirmed that there were clearly significant differences between *kf-1* null mice and the wild-type mice. The results of the elevated plus-maze test show that there was observed a significant decrease of exploratory locomotor activity on heights even though no significant differences were observed in acrophobia-like psychology. The results of the light/dark transition test indicate that the *kf-1* null mice prefer to stay in the dark compartment and that the activity in the dark compartment was not reduced. It has been concluded that there is an association between the increased anxiety-like behaviors and the increased ability of sensorimotor gating function of *kf-1* null mice observed in the prepulse inhibition test.

These behavioral patterns of *kf-1* null mice suggested the possibility that *kf-1* null mice could become a model animal for "social withdrawal disorder".

### 3. Deposition of Organism

The above-obtained mouse was deposited with RIKEN, the Institute of Physical and Chemical Research, BioResource Center as Reg_No.01916 (deposition date: October 12, 2006, address of the deposit authority: 3-1-1, Koyadai, Tsukuba-shi, Ibaraki-ken, Japan).

The details of the deposited mouse are shown below.
Reg_No.01916, systematic name: pKF1KO4.3.1, common-name and another strain name: Hicky mouse
(1) Genetic Classification: Targeted Mutation Congenic
(2) Origin of the strain and Generation Number: A mouse was created from a mouse embryonic stem cell having LoxP sequences inserted before and after the exon 4 of a mouse *kf-1* gene. The *kf-1* exon 4 was deleted by crossing with Cre Tg mice. The passage number: 18 generations
(3) Microbiological Breeding Environment: conventional
(4) Microbiological Characteristics: ICLAS (microscopic examination I, culture I, serum I) negative, S. aureus positive Detail of Lineage (characteristics and use): Exhibiting "social withdrawal"-like anxiety behaviors. Usable as a model animal for developing novel anxiolytic agents and antidepressants
(5) Breeding and Mating: Propagation effectiveness A
(6) Development Process of Lineage: Mating with C57BL/6N, the eighth generation
(7) Remark: Abnormal behaviors were observed. Typical "social withdrawal disorder"-like symptoms and anxiety-like behaviors were observed in the light/dark test and the elevated plus-maze test. In the forced swim test and the tail suspension test, so called mouse "depression" -like behaviors were not observed.
(8) Name of the Introduced Vector: pKF1KO-4.3
(9) Introduced Gene: Lox-pgk-Neo-*kf-1*[ex4]-LoxP, more precisely
   LoxP-pgk-Neo-*kf-1*[in3b]-*kf-1*[ex4a]-LoxP
   provided that the portion flanked by two LoxP cassettes, is deleted by crossing with Cre Tg mice, excepting a single LoxP site.
(10) Creation Method: ES cells
(11) Name of ES Cells of (10): 129SVJ
(12) Gene Detection Method: PCR
(13) Detail Of The Detection Method of (12) (primer sequence):
   20039FW: TCTTGGTTAAATAATGTATGCTCT
   17436FW: AACTTGAAGTCGCTGTCTTTTGG
   20292RV: CCCCTATAAAATTCTTTCCTATCC
   WT 277 bp, Hicky: 560 bp

## Claims

1. A vector for creating a *kf-1* gene knockout nonhuman animal exhibiting anxiety-like behaviors comprising:
LoxP-pM-M-*kf-1*[in3b]-*kf-1*[ex4a]-LoxP
wherein M is a selection marker gene,
pM is a promoter for expression of the selection marker gene,
*kf-1*[in3b] is a base sequence represented by SEQ ID NO: 2, and
*kf-1*[ex4a] is a base sequence represented by SEQ ID NO: 3.

2. A *kf-1* gene knockout nonhuman animal created by using the vector of claim 1, or a descendant thereof.

3. A modified nonhuman animal or a descendant thereof of claim 2, or a descendant of the modified nonhuman animal.

4. A method for screening of compounds useful for prevention, treatment or alleviation of an anxiety disorder or anxiety-like behaviors that is a part of depression or other psychiatric diseases,
the method comprising Steps (1) to (3) below:
(1) administering a test compound to the nonhuman animal or a descendant thereof of claim 2 or 3;
(2) measuring anxiety-like behaviors of the nonhuman animal in a group to which the test compound is administered and a group to which the placebo is administered; and
(3) selecting anxiolytic test compounds by comparing the results in Step (2).

5. A medical composition for preventing, treating or alleviating an anxiety disorder or anxiety-like behaviors that is a part of depression or other psychiatric diseases, the composition comprising the compound selected by the screening method of claim 4 as an active ingredient.
